(19) Europäisches Patentamt
European Patent Office
Office européen des brevets

(11) Publication number: **0 421 752 A2**

# (12) EUROPEAN PATENT APPLICATION

(21) Application number: **90310810.8**

(51) Int. Cl.[5]: **C07D 499/46**, A61K 31/43

(22) Date of filing: **03.10.90**

The title of the invention has been amended (Guidelines for Examination in the EPO, A-III, 7.3).

(30) Priority: **05.10.89 GB 8922411**
**01.08.90 GB 9016896**

(43) Date of publication of application:
**10.04.91 Bulletin 91/15**

(84) Designated Contracting States:
**AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **Ponsford, Roger John, SmithKline Beecham Pharm.**
**Brockham Park, Betchworth**
**Surrey RH3 7AJ(GB)**
Inventor: **Stachulski, Andrew Valentine, SmithKline Beecham Pharmaceuticals, Brockham Park, Betchworth**
**Surrey RH3 7AJ(GB)**

(74) Representative: **West, Vivien et al**
**Beecham Pharmaceuticals Great Burgh Yew Tree Bottom Road**
**Epsom Surrey KT18 5XQ(GB)**

## (54) Substituted acrylamido-penicillanic acid derivatives.

(57) Compounds of formula (I) and their derivatives:

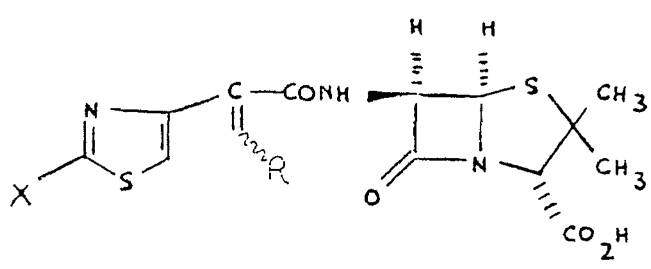

(I)

wherein X is hydrogen or a group $NHR^1$, wherein $R^1$ is hydrogen or an amino protecting group, and R is an optionally substituted spiro, fused or bridged bicyclic group optionally containing one or more heteroatoms selected from oxygen, nitrogen and sulphur, are new and useful in the treatment of bacterial infections in humans and animals.

EP 0 421 752 A2

## NOVEL COMPOUNDS

This invention relates to novel β-lactam containing compounds, their preparation and their use, and in particular to a novel class of penicillins. These compounds have antibacterial properties, and therefore are of use in the treatment of bacterial infections in humans and animals caused by a wide range of organisms.

US-A-3,622,569, US-A-4,416,880 and EP-A-0161617 disclose β-lactam antibiotics containing a substituted acrylamido side chain.

We have now found a particular class of penicillin antibiotics containing a substituted acrylamido side chain that possesses high antibacterial activity and a high level of stability to bacterial β-lactamases.

The present invention accordingly provides a compound of formula (I) or a pharmaceutically acceptable salt or in -vivo hydrolysable ester thereof:

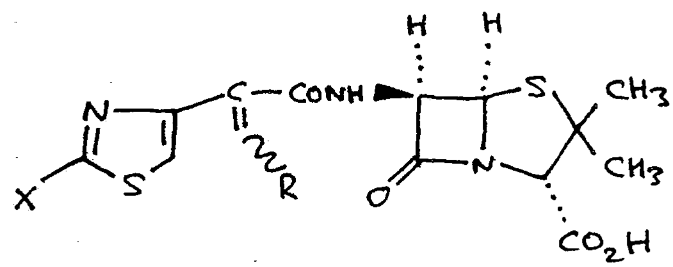

(I)

wherein X is hydrogen or a group $NHR^1$, wherein $R^1$ is hydrogen or an amino protecting group, and R is an optionally substituted spiro, fused or bridged bicyclic group optionally containing one or more heteroatoms selected from oxygen, nitrogen and sulphur.

The bicyclic group may contain from 6 to 12, preferably 6 to 10, ring atoms. Preferably each ring of the bicyclic system contains 3 to 6 ring atoms, one or more of which will be common to the other ring of the system.

Substituents for the bicyclic group include $C_{1-6}$ alkyl such as methyl, alkoxy such as $C_{1-6}$alkoxy, hydroxy and halogen.

One or other of the bicyclic ring systems may be unsaturated or aromatic.

Compounds of the invention may exist in two or more tautomeric forms, e.g. those having the partial structures below:

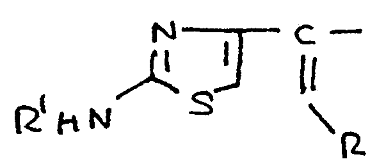

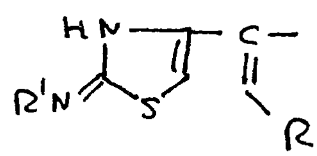

It should be understood that all tautomeric forms of the compound of formula (I) are included within the scope of the invention.

Suitable amino protecting groups $R^1$ are those well known in the art which may be removed under conventional conditions without disruption of the remainder of the molecule.

Examples of amino protecting groups $R^1$ include $C_{1-6}$ alkanoyl; benzoyl or benzyl optionally substituted in the phenyl ring by one or two substituents selected from $C_{1-4}$ alkyl, $C_{1-4}$ alkoxy, trifluoromethyl, halogen, or nitro; $C_{1-4}$ alkoxycarbonyl; benzyloxycarbonyl or trityl substituted as for benzyl above; allyloxycarbonyl, trichloroethoxycarbonyl or chloroacetyl.

Particularly preferred values of R within the present invention are the values set out hereinbelow in the Examples.

Preferably R is not attached via a tertiary carbon atom thereof.

As used herein, the term 'halogen' refers to fluorine, chlorine, bromine and iodine.

Examples of suitable pharmaceutically acceptable in vivo hydrolysable ester groups include those which break down readily in the human body to leave the parent acid or its salt. Suitable ester groups of this type include those of part formula (i), (ii), (iii) and (iv):

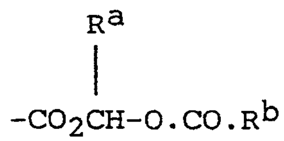

(i)

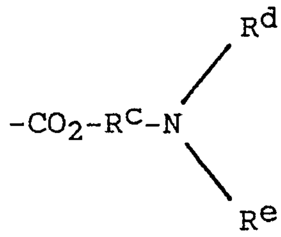

(ii)

$-CO_2CH_2-OR^f$ (iii)

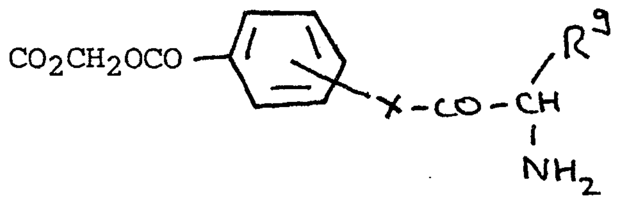

(iv)

wherein $R^a$ is hydrogen, $C_{1-6}$ alkyl, $C_{3-7}$ cycloalkyl, methyl, or phenyl, $R^b$ is $C_{1-6}$ alkyl, $C_{1-6}$ alkoxy, phenyl, benzyl, $C_{3-7}$ cycloalkyl, $C_{1-6}$ alkyl $C_{3-7}$ cycloalkyl, 1-amino $C_{1-6}$ alkyl, or 1-($C_{1-6}$ alkyl)amino $C_{1-6}$ alkyl; or $R^a$ and $R^b$ together form a 1,2-phenylene group optionally substituted by one or two methoxy groups; $R^c$ represents $C_{1-6}$ alkylene optionally substituted with a methyl or ethyl group and $R^d$ and $R^e$ independently represent $C_{1-6}$ alkyl; $R^f$ represents $C_{1-6}$ alkyl; $R^g$ represents hydrogen or phenyl optionally substituted by up to three groups selected from halogen, $C_{1-6}$ alkyl, or $C_{1-6}$ alkoxy; and X is (preferably o ) oxygen or (preferably o or p )NH.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; alkoxycarbonyloxyalkyl groups, such as ethoxycarbonyloxymethyl isopropoxy-carbonyloxy-methyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula:

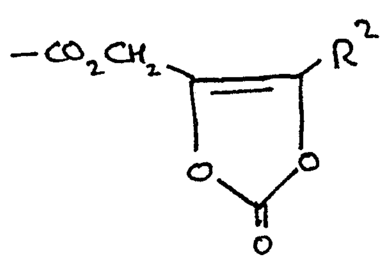

wherein $R^2$ is hydrogen, $C_{1-6}$ alkyl or phenyl.

The in-vivo hydrolysable esters of compounds of formula (I) have been found to be particularly well absorbed via the oral route.

Suitable pharmaceutically acceptable salts of the carboxy group of the compound of formula (I) include metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine,

dibenzylamine, N,N'-dibenzylethylenediamine, 1-ephenamine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins.

Some of the compounds of this invention may be crystallised or recrystallised from solvents such as methanol. In such cases solvates may be formed. This invention includes within its scope stoichiometric solvates including hydrates as well as compounds containing variable amounts of water that may be produced by processes such as lyophilisation.

Since the compounds of the formula (I) and their salts and in-vivo hydrolysable esters are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds of the formula (I) and their salts may be used for preparing the more pure forms used in the pharmaceutical compositions; these less pure preparations of the compounds of formula (I) and their salts should contain at least 1%, more suitably at least 5% and preferably from 10 to 49% of a compound of the formula (I) or salt thereof.

Compounds of the present invention may exist as either syn or anti isomers, or may exist as mixtures of syn and anti isomers containing at least 75% of one such isomer, or preferably at least 90% of one such isomer.

Herein the terms syn and anti refer to the configuration of the group R with respect to the carboxamido group, the syn-configuration (sometimes called the Z-configuration) being denoted thus:

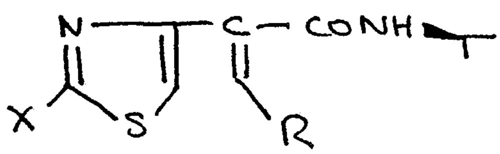

and the anti configuration (sometimes called the E-configuration) being denoted thus:

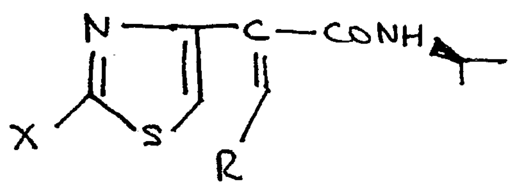

Preferred compounds of the present invention are the syn-isomers of the formula (IA):

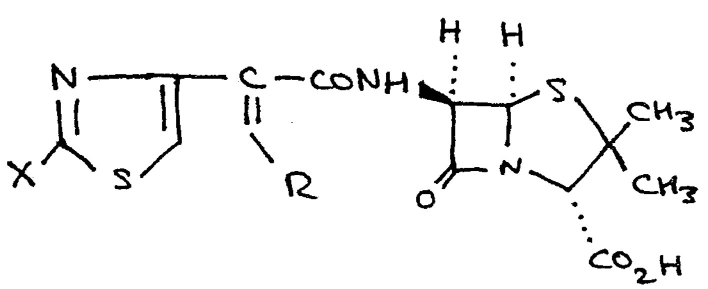

(IA)

wherein R and X are as hereinbefore defined.

The compounds of formula (I) may be prepared by treating a compound of formula (II) or salt thereof:

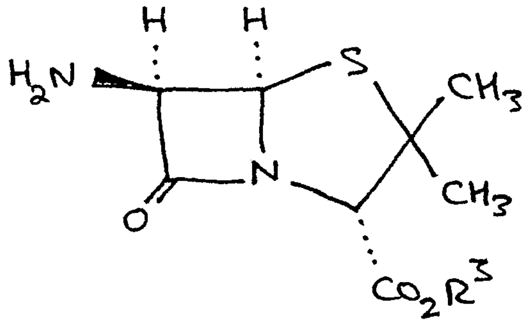

(II)

wherein the amino group is optionally substituted with a group which permits acylation to take place, and $R^3$ is hydrogen or a readily removable carboxyl blocking group; with an acylating agent derived from the acid of formula (III):

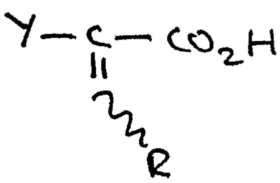

(III)

wherein Y is a group of formula:

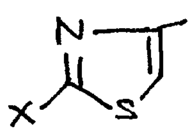

or a group which is convertable thereto, and R and X are as defined with respect to formula (I).

Any of the following reactions in any appropriate sequence may then be carried out:-

(i) removal of any amino-protecting group $R^1$;

(ii) removal of any carboxyl blocking group $R^3$;

(iii) formation of a pharmaceutically acceptable salt;

(iv) conversion of a carboxyl group into an ester function such as an in vivo hydrolysable ester.

(v) conversion of group Y to a group of formula

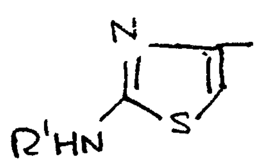

Suitable groups which permit acylation to take place and which are optionally present on the amino group of the starting material of formula (II) include N-silyl, N-stannyl and N-phosphorus groups, for example trialkylsilyl groups such as trimethylsilyl, trialkyltin groups such as tri-n-butyltin, groups of formula $-PR^aR^b$ wherein $R^a$ is an alkyl, haloalkyl, aryl, aralkyl, alkoxy, haloalkoxy, aryloxy, aralkoxy or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

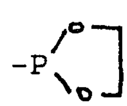

and

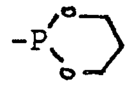.

Suitable carboxyl-blocking derivatives for the group $CO_2R^3$ in formula (II) include salts and ester derivatives of the carboxylic acid. The derivative is preferably one which may readily be cleaved at a later stage of the reaction. Suitable salts include metal salts, such as those with sodium, potassium and lithium, a preferred salt is sodium.

Suitable ester-forming carboxyl-blocking groups are those which may be removed under conventional conditions. Such groups for $R^3$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, t-butyl, t-amyl, diphenylmethyl (benzhydryl), triphenylmethyl, adamantyl,2-benzyloxyphenyl, 4-methylthiophenyl, tetrahydrofuran-2-yl, tetrahydropyran-2-yl, pentachlorophenyl, p-toluene-sulphonylethyl, methoxymethyl, a silyl, stannyl or phosphorus-containing group, such as described above, an oxime radical of formula $-N=CHR^4$ where $R^4$ is aryl or heterocyclic, or an in vivo hydrolysable ester radical such as defined above.

The carboxyl group may be regenerated from any of the above esters by usual methods appropriate to the particular $R^3$ group, for example, acid - and base -catalysed hydrolysis, or by enzymically - catalysed hydrolysis, or by hydrogenation under conditions wherein other parts of the molecule are unaffected.

A reactive N-acylating derivative of the acid of formula (III) is employed in the above process. The group $R^1$ in the acid of formula (III), when present, will be chosen such that the group $NHR^1$ does not react when the carboxy group in (III) is converted into the said N -acylating derivative. Thus, in many - although not all - of the suitable N -acylating derivatives of the acid (III) detailed below, $R^1$ cannot be hydrogen.

Suitable N-acylating derivatives of the acid (III) include acid (III) halides, preferably the acid chloride or bromide. Acylation with an acid halide may be effected in the presence of an acid binding agent, for example a tertiary amine (such as triethylamine or dimethylaniline), an inorganic base (such as calcium carbonate or sodium bicarbonate), molecular sieves (such as type 4 Angstroms) or an oxirane, which binds hydrogen halide liberated in the acylation reaction. The oxirane is preferably a ($C_{1-6}$)- 1,2-alkylene oxide - such as ethylene oxide or propylene oxide. The acylation reaction using an acid halide may be carried out at a temperature in the range -50°C to +50°C, preferably -20°C to +20°C, in aqueous or non-aqueous media such as aqueous acetone, aqueous tetrahydrofuran, ethyl acetate, dimethylacetamide, dimethylformamide (DMF), acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (III) with a halogenating (eg chlorinating or brominating) agent such as phosphorus pentachloride, thionyl chloride or oxalyl chloride.

Alternatively, the N-acylating derivative of the acid (III) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric or phosphorous acids) or aliphatic or aromatic sulphonic acids (such as methanesulphonic acid and p-toluenesulphonic acid respectively). When a symmetrical anhydride is employed, the acylation reaction may be carried out in the presence of an organic base such as 2,6-lutidine as catalyst.

When a mixed anhydride is employed the N-acylating derivative is preferably prepared in the presence of an organic base such as triethylamine and/or N,N-diisopropylethylamine in a suitable solvent such as DMF at between -50°C and room temperature. Alternatively, the N-acylating derivative may be prepared from an alkali metal salt of the acid of formula (III), such as the sodium salt, in a suitable solvent such as DMF at between -50°C and room temperature. The N-acylating derivative of the acid of formula (III) so derived may then be reacted with a compound of formula (II). The acylation reaction may conveniently be carried out at -50°C to +50°C in a suitable solvent such as water, acetonitrile or DMF at a temperature of not more than 0°C. The reaction may be carried out in the presence of a suitable base such as triethylamine or sodium hydrogen carbonate.

A further method of forming the N -acylating derivative of the acid of formula (III) wherein X is hydrogen or a protected $NH_2$, is to treat the acid of formula (III) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide,

preferably N,N-dimethylformamide. The N-acylating derivative of the acid of formula (III) so derived may then be caused to react with a compound of formula (II). The acylation reaction may conveniently be carried out at -40° to +30°C, if desired in the presence of an acid binding agent such as triethylamine. A catalyst such as 4-dimethylamino-pyridine may optionally also be added.

Other suitable acylating agents derived from the acid of formula (III) are thioesters of formula (IV)

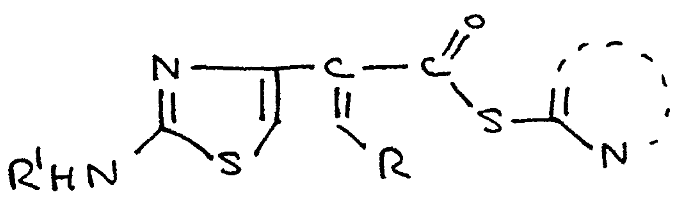

(IV)

wherein R and $R^1$ are as hereinbefore defined and

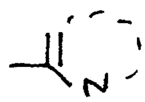

represents a 5- or 6-membered heterocyclic ring, which may contain, in addition to the nitrogen atom, one or two further heteroatoms, selected from oxygen, nitrogen and sulphur and which may be substituted or fused to a benzene ring which may itself be substituted.

Particular acylating agents derived from the acid of formula (III) are the thio esters (IVa) or (IVb)

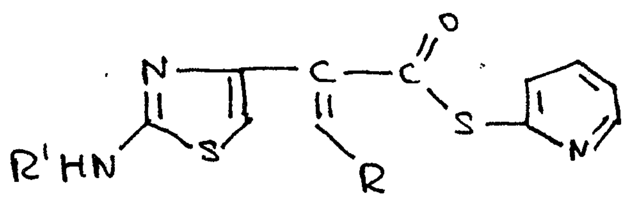

(IVa)

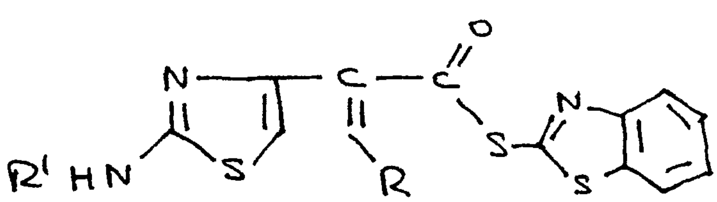

(IVb)

hydrogen.

Other suitable N -acylating derivatives of acid (III) include the acid azide; the activated esters derived from cyanomethanol; p-nitrophenol; 2,4-dinitrophenol; thiophenol; halophenols, including pentachlorophenol; monomethoxyphenol; N-hydroxy succinimide; N-hydroxybenzotriazole or 8-hydroxyquinoline; or include amides such as N-acylsaccharins, N-acylthiazolidin-2-thione or N-acylphthalimides; or an alkylidene iminoester prepared by reaction of the acid (III) with an oxime.

Other reactive N-acylating derivatives of the acid (III) include the reactive intermediates formed by reaction in situ with a condensing agent such as a carbodiimide, for example, N,N′-diethyl-, dipropyl- or diisopropylcarbodiimide, N,N′-di-cyclohexylcarbodiimide, or N-ethyl-N′[3-(dimethylamino)propyl]-carbodiimide; a suitable carbonyl compound, for example, N,N′-carbonyldiimidazole or N,N′-carbonylditriazole; an isoxazolinium salt, for example, N-ethyl-5-phenylisoxolinium-3-sulphonate or N-7-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

Compounds of formula (III) may be prepared by routes analogous to those disclosed in US-A-3,622,569, US-A-4,416,880 and EP-A-0 161 617.

In particular compounds of formula (III) may be prepared by condensation of an aldehyde of formula RCHO with an ester, such as the methyl ester, of the acid $YCH_2COOH$, wherein R and Y are as hereinbefore defined.

Any of the following reactions in any appropriate sequence may then be carried out:

(i) conversion of Y to a group of formula:

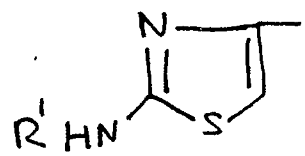

(ii) separation of E and Z isomers,

(iii) deamination of a group of formula:

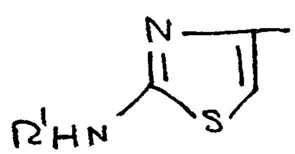

(iv) removal of a protecting group $R^1$ when present, and

(v) hydrolysis of an ester of the acid of formula (III).

The condensation reaction is typically carried out at low temperatures, e.g. about - 20°C, in the presence of a base such as piperidine, or by reflux with acetic acid-piperidine, catalysis in benzene or toluene and azeotropic removal of water.

Separation of E and Z isomers can be effected by chromatography, for example by silica gel chromatography in an ethyl acetate-hexane solvent system.

Processes for the deamination of an aminothiazole group to give a thiazole group are known and are described in J. Chem. Soc, 1973, 541, (J. Cadogan and G. Molina).

Advantageously, the removal of $R^1$, when $R^1$ is a protecting group such as N-acyl, and the hydrolysis of the ester group can be carried out in a single step, for example by refluxing with excess base, such as sodium hydroxide, in an inert solvent such as dioxan.

Y may be any suitable group, but it is preferred that Y be an $\alpha$-halo acetyl group (or an acetyl group which can be $\alpha$-halogenated), the conversion of which may be effected by condensation with optionally N-acylated thiourea. The condensation reaction is typically carried out at elevated temperatures in an inert solvent such as dimethyl formammide.

Aldehydes of formula RCHO are commercially available or can be obtained by oxidizing commercially available alcohols, using a suitable oxidizing agent such as pyridinium chlorochromate or dichromate.

Certain of the compounds of formula (III) are novel, and these novel compounds and their derivatives also form a part of the present invention.

The present invention also provides a pharmaceutical composition which comprises a compound of formula (I) or a pharmaceutically acceptable salt or in vivo hydrolysable ester thereof and a pharmaceutically acceptable carrier. The compositions of the invention include those in a form adapted for oral, topical or parenteral use and may be used for the treatment of the infection in mammals including humans.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral, topical or parenteral.

The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) is administered in the above-mentioned dosage range.

Compounds of the present invention are characterised by increased stability to $\beta$-lactamase producing organisms when compared to synthetic penicillins in commercial use such as amoxycillin.

The compound of the invention of formula (I) may therefore be used as the sole therapeutic agent in compositions of the invention or may be used in combination with other antibiotics or with a $\beta$-lactamase inhibitor.

Advantageously the compositions also comprise a compound of formula (V) or a pharmaceutically acceptable salt or ester thereof:

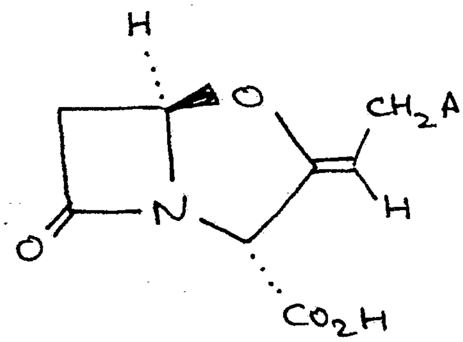

(V)

wherein A is hydroxyl; substituted hydroxyl; thiol; a group of formula $SO_2R^5$ wherein $R^5$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP O 053 893.

A further advantageous composition comprises an antibiotic compound according to the invention and a pharmaceutically acceptable carrier or excipient together with a $\beta$-lactamase inhibitor of formula (VI) or a pharmaceutically acceptable salt or in vivo hydrolysable ester thereof:

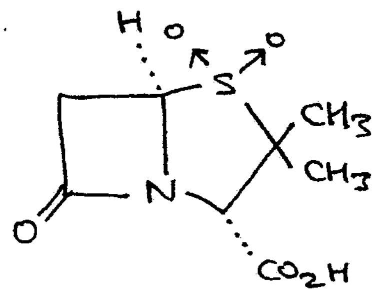

(VI)

wherein B is hydrogen, halogen or a group of formula:

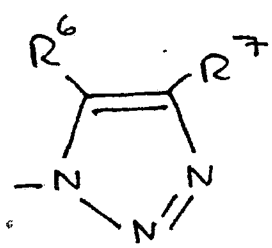

in which $R^6$ and $R^7$ are the same or different and each is hydrogen, $C_{1-6}$ alkoxycarbonyl, or carboxy or a pharmaceutically acceptable salt thereof.

Further suitable $\beta$-lactamase inhibitors include 6-alkylidene penem of formula (VII) below:

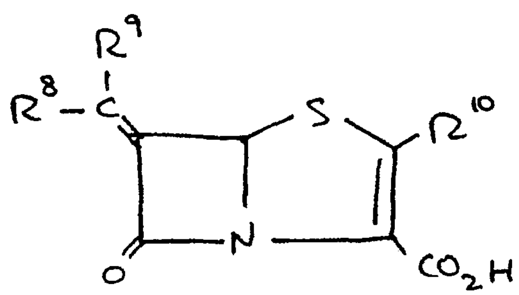

(VII)

or a pharmaceutically acceptable salt or in vivo hydrolysable ester thereof, wherein $R^8$ and $R^9$ are the same or different and each represents hydrogen, or a $C_{1-10}$ hydrocarbon or heterocyclic group optionally substituted with a functional group; and $R^{10}$ represents hydrogen or a group of formula $R^a$ or -$SR^a$ where $R^a$ is an optionally substituted $C_{1-10}$ hydrocarbon or heterocyclic group, as described in EP-A-0 041 768.

Other suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention.

Antibiotic compounds of the present invention are active against a broad range of bacteria, in particular they are useful for treatment of respiratory tract and urinary tract infections in humans and mastitis in cattle. It should be stressed that a particular advantage of certain compounds of the invention is their stability to β-lactamase enzymes and they are therefore effective against β-lactamase-producing organisms.

The antibiotic compounds of the present invention are active against both Gram-negative and Gram-positive organisms e.g. E.coli , including NCTC 10418, NCTC JT425 and 1077; respiratory pathogens such as H.influenzae , in particular Q1 and NEMC 1 and B.catarrhalis Ravasio; S.aureus such as Oxford, Russell and MB 9 and methicillin resistant strains such as V573; S.pyogenes such as CN10; S.agalactiae such as 2798; and S.pneumoniae such as PU7 and 1761.

The following Examples illustrate the preparation of the compounds of the present invention.

## Example 1

### Sodium 6β-[Z-[2-(2-aminothiazol-4-yl)-3-(bicyclo[2.2.1]hept-2-yl)]-propenamido]penicillanate, isomers

#### (a) 2-Norbornanemethanal

2-Norbornanemethanol (0.63g) in dry dichloromethane (5ml) was added, dropwise, to a stirring suspension of pyridinium chlorochromate (1.62g) in dry dichloromethane (10ml), at 0°C. After 2 hrs at ambient temperature the reaction appeared complete by t.l.c. The reaction mixture was taken up in ether and filtered through a silica gel pad. The filtrate was evaporated to a yellow oil which was flash chromatographed on silica gel, eluting with ethyl acetate-hexane mixtures, to give the desired product as a clear oil (0.335g). This was found to be an exo-endo mixture of isomers in the ratio 3:1. $\nu_{max}$ (neat) 1720cm$^{-1}$; δ($CDCl_3$, 60MHz), inter alia , 9.76 and 9.90 (1H, 2s, ca. 1:3) [K. Alder, G. Stein, and E. Rolland, Annalen , 1936, 525 , 247 (and refs. therein)].

#### (b) Methyl(E,Z)-[2-(2-acetamidothiazol-4-yl)-3(bicyclo[2.2.1]hept-2-yl)]propenoate

2-Norbornanemethanal (2.65g), methyl 2-acetamidothiazol-4-acetate (4.156g), piperidine (1.9ml) and

glacial acetic acid (1.1ml), were stirred in toluene (40ml), under reflux, with a water separator, for 25 hrs. By t.l.c the reaction was then near completion; the reaction mixture was allowed to cool and evaporated to low volume. The residue was taken up in ethyl acetate and washed three times with water, once with brine, dried, filtered and evaporated to a foam. This was flash chromatographed on silica gel, eluting with ethyl acetate-hexane mixtures. The Z isomer eluted from the column first as a semi-solid (0.659g) which was triturated under hexane and a little ether added. The resultant solid was filtered off to give the Z -isomer (0.393g), as a 3:1 mixture of exo and endo isomers, m.p. 166-168°C (from ethyl acetate-hexane); $\nu_{max}$ (KBr) 1709, 1658, and 1559cm$^{-1}$; $\delta$($CDCl_3$, 250MHz) 1.14-1.80 and 2.10-2.35 (10H, 2M), 2.22 (3H, s), 2.65, 2.97 (1H, 2m, ca . 3:1), 3.85, 3.86 (3H, 2s, ca . 1:3), 6.63, 6.79 (1H, 2d, J = 10.1, 9.9Hz, ca . 3:1), 6.91, 6.93 (1H, 2s, ca . 1:3), and 9.43 (1H,s, $D_2O$ exch). [Found: C, 60.3; H, 6.3; N, 8.6; S, 9.9. $C_{16}H_{20}N_2O_3S$ requires: C, 60.0; H, 6.3; N, 8.7; S, 10.0%]. Further elution of the column gave the E -isomer as a foam (0.74g); $\delta$($CDCl_3$, 60MHz), inter alia , 6.92 (1H,s) and 7.03 (1H, d, J = 10Hz).

(c) Z-[2-(2-aminothiazol-4-yl)-3-(bicyclo[2.2.1]hept-2-yl)]propenoic acid

Methyl Z-[2-(2-acetamidothiazol-4-yl)-3-bicyclo[2.2.1]hept-2-yl)]propenoate (0.320g) was dissolved in dioxan (3ml). 1M NaOH (5ml) was added and the mixture stirred at 95°C for 4h, when reaction appeared complete by t.l.c. The mixture was cooled and evaporated to dryness. The residue was taken up in water and washed twice with ether (each organic phase was backwashed with a little water). The combined aqueous phase was acidified with 5M HCl to pH4. The solid product obtained upon acidification was filtered and dried to give the desired material (0.210g) as a 3:1 mixture of exo and endo isomers: $\nu_{max}$ (KBr) 1690 (sh), 1629, 1559, and 1527cm$^{-1}$: $\delta$[$(CD_3)_2SO$, 250MHz], inter alia , 1.1-2.3 (10H, m), 6.33, 6.47 (1H, 2d, J = 10.3, 10.1Hz, ca . 3:1), 6.39, 6.41 (1H, 2s, ca . 1:3), 7.11 (2H, s, $D_2O$ exch), and 12.84 (1H, broad s, $D_2O$ exch). [Found: M , 264.0937. $C_{13}H_{16}N_2O_2S$ requires M , 264.0932].

(d) Sodium 6$\beta$-[[Z-2-(2-aminothiazol-4-yl)-3-(bicyclo[2.2.1]hept-2-yl)]propenamido]pencillanate

Z-[2-(2-aminothiazol-4-yl)-3-(bicyclo[2.2.1]hept-2-yl)]propenoic acid (0.208g) and 1-hydroxybenzotriazole monohydrate (0.127g) were stirred in dry DMF (2.5ml) at 0°C whilst N ,N ′-dicyclohexylcarbodi-imide (0.17g) was added. The mixture was allowed to warm to room temperature and stirred under argon for 2.75h. T.l.c. showed active ester to have formed. The mixture was filtered into a stirring solution of 6-aminopenicillanic acid (0.196g) and 1N NaOH (0.91ml) in $H_2O$ (2.5ml) (filtered solid was washed with fresh DMF). The mixture was stirred at room temperature for 2.5h. When complete by t.l.c., the reaction mixture was filtered, the filtrate evaporated to near dryness, taken up in water and adjusted to pH 8 with aqueous sodium hydrogen carbonate solution ($NaHCO_3$). This solution was washed twice with ethyl acetate: ether, 1:1 (backwashing each time with a little water). The combined aqueous phases were acidified to pH 2.5 with 5M HCl (aq) and extracted twice into ethyl acetate containing a little THF. The combined organic phases were washed twice with water, once with brine, dried, filtered and evaporated to a yellow solid (0.336g). This material was taken up in THF and chromatographed on silica gel, eluting with ethyl acetate:propan-2-ol:water mixtures. Product rich fractions were combined, evaporated to low volume, adjusted to pH 6.5 with $NaHCO_3$ (aq) and lyophilised to give the penicillin sodium salt (0.141g). By NMR this material contained 1-hydroxybenzotriazole (HOBt) and so was chromatographed on HP20SS, initially eluting with water to remove the HOBt and then with an increasing percentage of acetone to water. Product rich fractions were combined and evaporated to low volume. The pH was adjusted to 6.5 ($NaHCO_3$) and the solution lyophilised to give the pure penicillin sodium salt (0.110g) as a mixture of diastereoisomers; $\nu_{max}$ (KBr) 1763, 1660 (sh), 1609, and 1525cm$^{-1}$; $\delta$($D_2O$, 250MHz) 0.80-2.40 (10H, m), 1.56 (6H, m), 2.33, 2.72, (1H, 2m, ca . 3:1), 4.22 (1H, s), 5.60 (2H, m), 6.20, 6.38 (1H, 2m, ca , 3:1) and 6.43 (1H, s); m/e 463 (MH-Na + N$^+$), and 485 (MH$^+$).

Example 2

Sodium 6$\beta$-[Z-[2-(2-aminothiazol-4-yl)-3-(3-methylbicyclo[2.2.1]hept-2-yl)]propenamido]penicillanate, isomers

(a) 3-Methyl-2-norbornanemethanal

3-Methyl-2-norbornanemethanol (4.386g) was oxidized using pyridinium chlorochromate as described in Example 1(a) [reaction proceeded for 2.5hrs]. The oil thus obtained was distilled (Kugelrohr) to give the desired product as a clear oil (3.66g) which was found to be a 1:1 exo-endo mixture; $\nu_{max}$ (film) 1720 and 1700(sh)$cm^{-1}$; δ($CDCl_3$,60MHz), inter alia , 9.79 and9.93 (1h,2d). (O.Diels, K. Alder et al., Annalen , 1929, 470 , 62).

(b) Methyl (E,Z)-[2-(2-acetamidothiazol-4-yl)-3-(3-methylbicyclo[2.2.1]hept-2-yl)]propenoate

3-Methyl-2-norbornanemethanal (1.40g) was condensed with methyl 2-acetamidothiazol-4-acetate (2.14g) as described in Example 1(b) [reaction proceeded for 30hrs]. Following chromatography on silica gel, the Z-isomer eluted first as an oil which solidified on trituration with hexane and a little ether. The resultant solid was filtered off to give the Z-isomer (0.308g), as a 2:1 mixture of exo and endo isomers (n.m.r.), mp. 163-166°C and 183-185°C (from ethyl acetate-hexane); $\nu_{max}$ (KBr) 1718, 1654, and 1560$cm^{-1}$; δ($CDCl_3$,400MHz), inter alia , 0.93, 0.99 (3H,2d,J=6.9,7.0Hz), 2.22, 2.23 (3H,2s), 3.85 (3H,s), 6.57, 6.72 (1H,2d,J=10.7,10.6Hz), 6.87, 6.89 (1H,2s), and 9.15(1H,br s,$D_2O$ exch.) (Found:C,61.5; H,6.8; N,8.3; S,9.3. $C_{14}H_{22}N_2O_3S$ requires: C,61.0; H,6.3; N,8.4; S,9.6%). Further elution of the column gave the E-isomer (0.36g) as an oil; δ($CDCl_3$,60MHz), inter alia , 6.95 (1H,s) and 7.10 (1H,d,J=10Hz).

(c) Z-[2-(2-Aminothiazol-4-yl)-3-(3-methylbicyclo[2.2.1]hept-2-yl)]propenoic acid

Methyl Z-[2-(2-acetamidothiazol-4-yl)-3-(3-methylbicyclo[2.2.1]hept-2-yl)]propenoate (0.217g) was hydrolysed as described in Example 1(c) [reaction proceeded for ca 4hrs]. The solid material obtained upon acidification to pH 3.5 was filtered off, washed with water and dried to give the desired product (0.147g) as a 2:1 mixture of exo and endo isomers: $\nu_{max}$ (KBr) 1690(sh), 1627, 1559, and 1528$cm^{-1}$: δ[$(CD_3)_2SO$, 400MHz], inter alia , 0.88, 0.93 (3H,2d,J=6.9Hz), 1.03-2.32 (10H,m), 6.27, 6.40 (1H,2d,J=10.7,10.5Hz], 6.36, 6.37 (1H,2s), and 7.11 (2H,br s,$D_2O$ exchanged); m/e 278($MH^+$).

(d) Sodium 6β-[Z-[2-(2-aminothiazol-4-yl)-3-(3-methylbicyclo[2.2.1]hept-2-yl)]propenamido]penicillanate, isomers

Z-[2-(2-aminothiazol-4-yl)-3-(3-methylbicyclo[2.2.1]hept-2-yl)]propenoic acid (0.140g) was coupled to 6-aminopenicillanic acid (0.125g), as described in Example 1(d) [reaction proceeded for 5hrs at ambient temperature and a further 15hrs at 0°C]. When the aqueous phase was acidified to pH3 a pale solid precipitated which was filtered off, washed with water and dried to give impure product (0.155g). This was taken up in THF and chromatographed on silica gel eluting with ethyl acetate: propan-2-ol:water mixtures. Appropriate fractions were combined,concentrated, adjusted to pH7 using aqueous $NaHCO_3$ and lyophilised to give the title penicillin (0.076g); $\nu_{max}$ (KBr) 1767, 1660(sh), 1610, and 1525$cm^{-1}$; δ($D_2O$,250MHz) 0.70-1.05 (3H,m), 1.05-2.30 (10H,m), 1.49, 1.59, 1.61 (6H,3s), 4.19 (1H,s), 5.53-5.71 (2H,m), 6.18, 6.34 (1H,2d,J=10.8Hz, ca .2:1), and 6.41 (1H,s); m/e 499 ($MH^+$) and 477 ($M-Na+H^+$). Further elution of the column gave additional, less pure penicillin (0.048g).

Example 3

Sodium 6β-[Z-[2-(2-aminothiazol-4-yl)-3-(bicyclo[2.2.1]hept-5-en-2-yl)]propenamido]penicillanate, isomers

(a) Methyl (E,Z)-[2-(2-acetamidothiazol-4-yl)-3-(bicyclo[2.2.1]hept-5-en-2-yl)]propenoate

Bicyclo[2.2.1]hept-5-ene-2-carboxaldehyde (1.22g) was condensed with methyl 2-acetamidothiazol-4-acetate (2.14g) as described in Example 1(b) [reaction proceeded for 18hrs]. Following chromatography on silica gel, the Z -isomer eluted first as a solid (0.787g), which was recrystallized from ethyl acetate-hexane

to give the Z-isomer (0.605g) in two crops as 2:1 (0.471g) and 1:1 (0.134g) endo:exo mixtures (n.m.r.), m.p. 147-149°C and 158-160°C; $\nu_{max}$(KBr) 1707, 1646, 1563(br) and 1497(wk)cm$^{-1}$; $\delta$($CDCl_3$, 400MHz), inter alia (major isomer (endo)), 0.9 (1H, ddd, J =2.5, 4.0, 11.9Hz), 1.35 (1H, br d, J =8.3Hz), 1.48 (1H, br dd, J =2.0, 8.2Hz), 2.1 (1H, ddd, J =3.7, 9.1, 11.8Hz), 2.9 (1H, br s), 2.95 (1H, br s), 3.25 (1H, tt, J =3.8, 9.8Hz), 3.87 (3H, s), 6.02 (1H, dd, J =2.8, 5.6Hz), 6.25 (1H, dd, J =3.0, 5.6Hz), 6.34 (1H, d, J =10.4Hz), 6.89 (1H, s), and 9.42 (2H, br s), inter alia (minor component (exo)) 6.13 (2H, m), 6.73 (1H, d, J =10.4Hz), and 6.94 (1H, s) (Found: C, 60.7; H, 6.1; N, 8.7; S, 10.7. $C_{16}H_{18}N_2O_3S$ requires: C, 60.4; H, 5.7; N, 8.8; S, 10.1%). Further elution of the column gave the E-isomer (0.977g) as an oil; $\delta$($CDCl_3$, 90MHz), inter alia , 6.58 and 6.98 (1H, 2d, J =10Hz, ca . 3:2) and 6.84 + 6.86 (1H, 2s, ca . 2:3).

(b) Z-[2-(2-aminothiazol-4-yl)-3-(bicyclo[2.2.1]hept-5-en-2-yl)]propenoic acid

Methyl Z-[2-(2-acetamidothiazol-4-yl)-3-(bicyclo[2.2.1]hept-5-en-2-yl)]propenoate (0.430g), isomers, ca . 1:1 exo:endo, was hydrolysed as described in Example 1(c) [reaction proceeded for 5hrs]. The solid material obtained upon acidification to pH3.5 was spun down in a centrifuge, washed twice with water, and dried to give the desired product (0.248g) as an endo:exo mixture ca 4:3: $\nu_{max}$ (KBr) 1685(sh), 1628, 1565, and 1528cm$^{-1}$; $\delta$[$(CD_3)_2SO$, 250MHz], 0.68-0.78 (1H, m), 1.2-1.5 (3H, m), 1.9-3.17 (3H, m), 5.99 (1H, d, J =10.6Hz), 5.95-6.05 and 6.24-6.32 (2H, 2m, endo), 6.14 (2H, m, exo), 6.35, 6.42 (1H, 2s, exo + endo) and 7.09 (2H, br s, $D_2O$ exch); m/e 263 ($MH^+$).

(c) Sodium 6β-[Z-[2-(2-aminothiazol-4-yl)-3-(bicyclo[2.2.1]hept-5-en-2-yl)]propenamido]penicillanate, isomers

Z-[2-(2-aminothiazol-4-yl)-3-(bicyclo[2.2.1]hept-5-en-2-yl)]propenoic acid (0.245g) was coupled to 6-aminopenicillanic acid (0.232g), as described in Example 1(d). When the aqueous phase was acidified to pH3.5 a pale solid precipitated. This was spun down in a centrifuge, washed well with water, (supernatant decanted each time), and dried to give impure product (0.300g). This material was chromatographed on silica gel, eluting with ethyl acetate: propan-2-ol: water mixtures. Appropriate fractions were combined, concentrated, adjusted to pH6.5 with $NaHCO_3$(aq) and lyophilized. The solid thus obtained (0.29g) contained some HOBt and so was rechromatographed on HP20SS, eluting with increasing percentages of acetone in water. Product rich fractions were combined, concentrated and lyophilized to give the title penicillin (0.125g) as a 1:1 mixture of exo and endo isomers (NMR); $\nu_{max}$(KBr) 1767, 1655(sh), 1609 and 1526cm$^{-1}$; $\delta$($D_2O$, 250MHz) 0.74-0.87, 2.63-2.74 (1H, 2m), 1.20-1.68 (4H, m), 1.50, 1.61 (6H, 2s), 1.50-2.13, 2.13-2.33 (1H, 2m), 2.81-3.01 (2H, m), 4.21, 4.22, 4.23, 4.23 (1H, 4s), 5.52-5.67 (2H, m), 5.82-6.35 (3H, m), 6.42 and 6.45 (1H, 2s); m/e 461 ($M\text{-}Na+H^+$). Further elution of the column gave additional, less pure penicillin (0.031g).

Example 4

Sodium 6β-[Z-[2-(2-aminothiazol-4-yl)-3-(bicyclo[4.4.0]dec-2-yl)]propenamido]penicillanate, isomers

(a) Methyl 1-naphthoate

1-Naphthoic acid (25g) and 4-toluenesulphonic acid (2.5g)were heated at reflux in methanol (200ml) for 72h. The bulk of the methanol was removed by evaporation, then the residue was neutralised with half-saturated aqueous $NaHCO_3$ and extracted once with ether:ethyl acetate, 1:1 and once with ether. The combined organic extracts were washed with $NaHCO_3$(aq.), water and brine, then dried and evaporated to an oil which was distilled in vacuo to give the ester (22.8g), b.p. 105-120°C/0.3mm.Hg; $\delta$($CDCl_3$, 90MHz) 3.95 (3H,s), 7.30-8.20 (6H,m), and 8.87 (1H, m).

(b) Methyl bicyclo[4.4.0]decane-2-carboxylate

Methyl 1-naphthoate (5g) in glacial acetic acid (40ml) was hydrogenated at ambient temperature and

pressure in the presence of 5% rhodium on carbon (1.25g). After a total of 96h, with three changes of catalyst, no starting material was visible by t.l.c. The catalyst was filtered and washed with acetic acid, then the combined filtrate was evaporated to near dryness and diluted with ether. The resulting solution was washed with aq. $NaHCO_3$ (2x), water, and brine, then dried and evaporated to an oil which was subjected to Kugelrohr distillation, giving the title compound as a colourless oil (3.87g); δ($CDCl_3$, 250MHz), 1.05-1.90 (15H, m), 2.10 (1H, m), 2.46 (1H, m), and 3.66 (3H, s).

(c) Bicyclo[4.4.0]decane-2-carboxaldehyde

Methyl bicyclo[4.4.0]decane-2-carboxylate (1.96g) in anhydrous toluene (15ml) was stirred under argon at -90°C and treated with 1.5M di-isobutylaluminium hydride in toluene (6.7ml). After 1.5h at the same temperature, the reaction was worked up by adding methanol (1ml), then, after being allowed to regain ambient temperature, saturated aqueous sodium sulphate (20ml). The mixture was filtered, the precipitate was washed with ether and water, then the organic phase was separated, washed with water (2x) and brine, then dried. Evaporation gave an oil which was chromatographed on silica, eluting with ethyl acetate-hexane mixtures. Appropriate fractions were combined and evaporated to an oil (1.14g) which by n.m.r. analysis was a mixture of the desired aldehyde plus unreacted methyl ester. The aldehyde component showed δ - ($CDCl_3$, 90MHz), *inter alia* , 9.62 (br s); the mixture could be used directly in the next step.

(d) Methyl (E,Z)-[2-(2-acetamidothiazol-4-yl)-3-bicyclo[4.4.0]dec-2-yl)]propenoate, isomers

Bicyclo [4.4.0]decane-2-carboxaldehyde from part (c) (1.14g, contaminated with methyl ester) was condensed with methyl 2-acetamidothiazol-4-acetate (0.86g) as described in Example 1(b), for a total of 94h. Workup and chromatography as described therein afforded firstly the Z-ester as an exo-endo mixture (0.213g) (Found: *M* , 362.1676. $C_{19}H_{26}N_2O_3S$ requires *M* , 362.1664); $\nu_{max}$(KBr) 1723, 1698 (sh), 1669(sh), 1550, and 1435cm$^{-1}$; δ($CDCl_3$, 250MHz) 1.10-1.90 (16H, m), 2.23 (3H, s), 2.69, 2.94 (1H, 2m, *ca* . 4:3), 3.87 (3H, s), 6.51, 6.71 (1H, 2d, *J* =10Hz, *ca* . 3:4), 6.89 (1H, s), and 9.10 (1H, br s, $D_2O$ exch). Further elution gave the E-ester as an exo-endo mixture (0.206g) which showed δ($CDCl_3$, 90MHz), *inter alia* , 6.76, 6.82 (1H, 2s), 6.93 and 7.06 (1H, 2d *J* s=10Hz). In this series neither isomer could be crystallised.

(e) Z-[2-(2-Aminothiazol-4-yl)-3-(bicyclo[4.4.0]dec-2-yl)]propenoic acid, isomers

Methyl Z-[2-(2-acetamidothiazol-4-yl)-3-(bicyclo[4.4.0]dec-2-yl)]propenoate (0.205g) was hydrolysed as described in Example 1(c) using 1M NaOH (2.85ml). The solid obtained on acidification to pH4 was filtered, washed with water and dried to give the title acid (0.131g); $\nu_{max}$(KBr) 1695(sh), 1624, 1559, 1527, and 1445cm$^{-1}$; δ[$(CD_3)_2SO$, 250MHz] 1.00-1.80 (16H, m), 2.40-2.85 (1H, 2m), 6.22, 6.40 (1H, 2d, *J* s=10Hz), 6.36 (1H, s), and 7.06 (2H, br s, $D_2O$ exch) (Found: *M* , 306.1405. $C_{16}H_{22}N_2O_5$ requires *M* , 306.1402).

(f) Sodium 6β-[Z-[2-(2-aminothiazol-4-yl)-3-(bicyclo[4.4.0]dec-2-yl)]propenamido]penicillanate, isomers

Z-[2-(2-Aminothiazol-4-yl)-3-(bicyclo[4.4.0]dec-2-yl)]propenoic acid (0.121g) was coupled to 6-aminopenicillanic acid (0.095g) as described in Example 1(d). Coupling was allowed to proceed for 2.5h. The solid obtained on acidification to pH3.5 was filtered, washed with water, and dried to give impure product (0.165g). This was purified by chromatography on silica gel, eluting with ethyl acetate: propan-2-ol-: water mixtures. Appropriate fractions were combined, concentrated, adjusted to pH7 using aq. $NaHCO_3$ and freeze-dried to give the title penicillin (0.128g); $\nu_{max}$(KBr) 1768, 1655(sh), 1609, and 1522cm$^{-1}$; δ[$(CD_3)_2SO$,250MHz] 1.00-2.00 (16H, m), 1.47, 1.52, 1.54 (6H, 3s), 2.30-2.70 (1H, m), 3.92 (1H, br s), 5.35-5.55 (2H, m, 2ABq on $D_2O$ exch.), 5.95-6.25 (1H, 2d, *J* s=10Hz), 6.19 (1H, s), 7.05 (2H, br s, $D_2O$ exch.), 8.45, and 8.87 (1H, 2d, $D_2O$ exch.); m/e 527 ($MH^+$) and 505 ($MH^+$ of free acid).

Example 5

Sodium 6β-[Z-[2-(2-aminothiazol-4-yl)-3-(bicyclo[4.3.0]non-7-yl)]propenamido]penicillanate, isomers

(a) Bicyclo[4.3.0]nonan-7-one

1-Indanone (4.09g) was dissolved in glacial acetic acid (30ml) and hydrogenated at ambient temperature and pressure in the presence of 5% rhodium on carbon (1g). Further catalyst (0.8g) was added after 19h; after a total of 42h no starting material was visible by t.l.c. Workup as described in Example 4(b) afforded an oil (1.55g) which by i.r. and n.m.r. analysis was a mixture of the desired ketone plus the corresponding secondary alcohol. This material in acetone (10ml) was stirred at 20°C and treated dropwise with Jones' reagent (0.7M $CrO_3$ in dil. $H_2SO_4$) until a permanent orange coloration resulted. Excess oxidant was destroyed by addition of propan-2-ol, then the reaction was diluted with water and extracted twice with pentane. The combined organic extracts were washed with water and brine, dried, evaporated and finally distilled (Kugelrohr) to afford the title ketone as a colourless oil (1.29g); $\nu_{max}$ (film) 3460(w), 1730, and 1440cm$^{-1}$; δ($CDCl_3$, 250MHz) 1.00-2.40 (14H, m); m/e 138($M^+$). (H.O. House et al , J.Org.Chem ., 1963, 28 , 31 and references therein).

(b) (E,Z)-7-(methoxymethylene)bicyclo[4.3.0]nonane, isomers

(Methoxymethyl)triphenylphosphonium chloride (3.42g) was suspended in anhydrous tetrahydrofuran (THF) (10ml) and stirred under argon at 0°C while a solution of potassium t -butoxide (1.12g) in THF (10ml) was added dropwise. After 0.5h, bicyclo[4.3.0]nonan-7-one (1.29g) was added via a syringe to the orange-red solution and the mixture was allowed to regain ambient temperature. After 4h, the then pale yellow solution was diluted with water and extracted with ether. The organic phase was washed with water (2x), brine, dried, evaporated and flash-chromatographed on silica gel, being applied in toluene solution and eluted with ethyl acetate-hexane mixtures. Product-rich fractions (t.l.c.) were combined, evaporated, and twice distilled (Kugelrohr) to afford the enol ether (1.28g); $\nu_{max}$(film) 1740(m), 1690, and 1445cm$^{-1}$; δ($CDCl_3$, 90MHz) 0.80-2.70 (14H, m), 3.39, 3.42, 3.48 (3H, 3s), and 5.55-5.85 (1H, m); m/e 166 ($M^+$, 62%), and 134 (M-MeOH$^+$, 95%) (Found: M , 166.1361. $C_{11}H_{18}O$ requires M , 166.1358). This mixture of isomers was progressed directly.

(c) Bicyclo[4.3.0]nonane-7-carboxaldehyde, isomers

(E,Z)-7-(Methoxymethylene)bicyclo[4.3.0]nonane (1.25g) in THF (7ml) and water (3ml) was stirred with toluene- 4-sulphonic acid, hydrate (1.90g) at ambient temperature for 2.25h, then stored at -10°C for 16h. Aqueous $NaHCO_3$ (15ml) was added and the mixture extracted with pentane. The organic phase was separated, washed with water (2x), brine, dried and evaporated to give essentially pure aldehyde (1.15g); $\nu_{max}$(film) 2700, 1725, and 1425cm$^{-1}$; δ($CDCl_3$, 90Hz) 0.80-3.00 (15H, m), 9.53, 9.66, and 9.75 (1H, 3d). Comparison with literature data (P.H. Lewis, S. Middleton, M.J. Rosser, and L.E. Stock, Aust.J.Chem. , 1980, 33 , 1049) suggested that the major isomer possessed the cis -ring junction.

(d) Methyl (E,Z)-[2-(2-acetamidothiazol-4-yl)-3-(bicyclo[4.3.0]non-7-yl)]propenoate, isomers

Bicyclo[4.3.0]nonane-7-carboxaldehyde (1.15g) and methyl 4-chloroacetoacetate (1.20g) were stirred at -10°C under argon with piperidine (2 drops). The temperature was allowed to rise to 0-5°C, then further piperidine and acetic acid (5 drops each) were added. After a total of 5.5h, the mixture was diluted with ether, washed with water (3x) and brine, dried and evaporated to an oil (2.24g). Without purification, this material was dissolved with N -acetylthiourea (0.94g) in anhydrous DMF (4ml) and heated at 85-90°C for 2h in the presence of 4A molecular sieves. After this time the dark solution was diluted with ethyl acetate, washed with water (4x) and brine, dried and evaporated to an oil (2.06g). Chromatography on silica gel, eluting with ethyl acetate-hexane mixtures, afforded firstly the Z-ester (0.63g), which slowly crystallized from a small volume of ethyl acetate-hexane, m.p. 110-111°C (twice crystallized) (Found: C, 62.3; H, 7.0; N, 8.0; S, 9.1. $C_{18}H_{24}N_2O_3S$ requires C, 62.1; H, 6.9; N, 8.0; S, 9.2%); δ($CDCl_3$, 250MHz) 0.80-2.10 (14H, m), 2.19 (3H, s), 2.64, 3.10 (1H, 2m), 3.84, 3.86 (3H, 2s), 6.55, 6.58 (1H, 2m, both J s=10Hz), 6.91, 6.92 (1H, 2s), and 10.04 (1H, br s, $D_2O$ exch). This material was a mixture of isomers in a 2:1 ratio. Further elution of the

column afforded the E-esters (0.22g) which showed δ($CDCl_3$, 90MHz), inter alia , 6.77 (1H, s), and 6.94 (1H, d, J =10Hz).

(e) Z-[2-(2-Aminothiazol-4-yl)-3-(bicyclo[4.3.0]non-7-yl)]propenoic acid, isomers

Methyl Z-[2-(2-acetamidothiazol-4-yl)-3-(bicyclo[4.3.0]non-7-yl)]propenoate (0.300g) was hydrolysed as described in Example 1(c) using 1M NaOH (4.3ml). The solid obtained on acidification to pH4 was filtered, washed with water, and dried to give the title acid as a 2:1 mixture of isomers (0.220g); $\nu_{max}$(KBr) 1680(sh), 1620, 1559, and 1528cm$^{-1}$; δ[$(CD_3)_2SO$, 250MHz] 0.75-2.05 (14H, m), 2.99 (0.3H, m), 6.28, 6.32 (1H, 2d, both J s=10Hz), 6.37, 6.38 (1H, 2s), and 7.06 (2H,br s, $D_2O$ exch); m/e 292 ($M^+$) (Found; M , 292.1243. $C_{15}H_{20}N_2O_2S$ requires M , 292.1245).

(f) Sodium 6β-[Z-[2-(2-aminothiazol-4-yl)-3-(bicyclo[4.3.0]non-7-yl)]propenamido]penicillanate, isomers

Z-[2-(2-Aminothiazol-4-yl)-3-(bicyclo[4.3.0]non-7-yl)]propenoic acid (0.200g) was coupled to 6-amino penicillanic acid (0.162g) as described in Example 1(d). Coupling proceeded for 3h; the solid material obtained on acidification to pH4 was filtered, washed with water, and dried to afford crude product (0.266g). Chromatography as described in Example 4(f) afforded the title penicillin (0.159g); $\nu_{max}$(KBr) 1767, 1655(sh), 1609, 1526, and 1505(sh)cm$^{-1}$; δ[$(CD_3)_2SO$ + $D_2O$, 1:1, 250MHz] 0.95-2.30 (14H, m), 1.67, 1.76 (6H, 2s), 2.55, 3.00 (1H, 2m), 4.27, 4.28 (1H, 2s), 5.65-5.75 (2H, m), 6.25-6.40 (1H, m), 6.52, and 6.53 (1H, 2s); m/e 513 ($MH^+$) and 491 ($MH^+$, free acid). The material was still a 2:1 isomeric mixture by n.m.r.

## Example 6

### Sodium 6β-[Z-[2-(2-aminothiazol-4-yl)-3-(7-oxabicyclo[2.2.1]hept-2-yl)]propenamido]penicillanate

(a) Endo-7-oxabicyclo[2.2.1]heptane-2-carboxaldehyde

Methyl endo 7-oxabicyclo[2.2.1]heptane-2-carboxylate (1.04g; W.L. Nelson, D.R. Allen, and F.F. Vicenzi, J.Med.Chem ., 1971, 14 , 698; W.L. Nelson and D.R. Allen, J.Het.Chem ., 1972, 9 , 561) was stirred in anhydrous dichloromethane (10ml) under argon at -90°C and treated with 1.0M di-isobutylaluminium hydride in hexane (6.7ml). After 2h at the same temperature, methanol (1ml) was added, followed by a solution of sodium sulphate (2g) in water (20ml). The mixture was allowed to regain room temperature, filtered and washed with ether and water. The organic phase was separated then the aqueous was saturated with sodium chloride and extracted with ether (2x) and dichloromethane (3x). The total organic extracts were thoroughly dried over anhydrous magnesium sulphate with stirring, then evaporated to give the aldehyde (0.81g) containing just a trace of unreduced ester; $\nu_{max}$(film) 1715, 1470(m), and 1450(m)cm$^{-1}$; δ($CDCl_3$, 90MHz) 1.40-2.00 (6H, m), 3.05 (1H, m), 4.55-4.90 (2H, m), and 9.71 (1H, d, J =2Hz); m/e 127 ($MH^+$, electron impact) and 144 [$MNH_4^+$, chemical ionisation (ammonia)].

(b) Methyl Z-[2-(2-acetamidothiazol-4-yl)-3-(7-oxabicyclo[2.2.1]hept-2-yl)]propenoate

Endo -7-oxabicyclo[2.2.1]heptane-2-carboxaldehyde (0.81g) and methyl 4-chloroacetoacetate (0.90g) were dissolved in ethyl acetate (15ml) and stirred at 0°C in the presence of 4A molecular sieves. Glacial acetic acid (0.36ml) and piperidine (0.6ml) were added and stirring continued for 1h. The solution was allowed to regain ambient temperature, diluted with ethyl acetate, washed with water (3x) and brine, dried and evaporated to a oil (1.25g). Without purification this material was dissolved with N -acetylthiourea (0.59g) in DMF (4ml) and heated at 85-90°C for 2h in the presence of 4A molecular sieves. The reaction mixture was cooled, diluted with ethyl acetate:ether, 1:1 and washed with water (8x), then with brine, dried and evaporated to an oil (0.83g). Chromatography on silica gel, eluting with ethyl acetate-hexane mixtures, afforded on evaporation of appropriate fractions the title ester (0.053g); δ($CDCl_3$, 250MHz) 1.50-2.20 (6H, m), 2.28 (3H, s), 3.09 (1H, m), 3.87 (3H, s), 4.41 (1H, approx.d, J=4Hz), 4.69 (1H, approx.t), 6.77 (1H, d,

J = 10Hz), 6.97 (1H, s), and 9.80 (1H, br s); this product appeared to be very largely one isomer, probably endo, with just a trace of the other isomer (δ6.71, d, J = 10Hz); m/e 322 ($M^+$).

(c) Z-[2-(2-Aminothiazol-4-yl)-3-(7-oxabicyclo[2.2.1]hept-2-yl]propenoic acid

Methyl Z-[2-(2-acetamidothiazol-4-yl)-3-(7-oxabicyclo[2.2.1]hept-2-yl)]propenoate (0.052g) was hydrolysed using 1M NaOH (0.80ml) as described in Example 1(c). Following acidification to pH2.0, the aqueous phase was saturated with sodium chloride and extracted with ethyl acetate: THF, 3:1 (3x), then once with THF. The total extract was rigorously dried over magnesium sulphate and evaporated to crude product (0.051g) which was purified by chromatography on silica gel, eluting with ethyl acetate:propan-2-ol: water mixtures. Pooling and evaporation of appropriate fractions afforded the acid (0.023g); δ[$(CD_3)_2SO$, 250MHz)] 1.20-1.90 (6H, m), 2.89 (1H, m), 4.20, 4.55 (2H, 2m), 6.24 (1H, d, J = 10Hz), 6.46 (1H, s), and 6.95 (2H, br s, $D_2O$ exch); m/e 267 ($MH^+$). This material was still largely one isomer.

(d) Sodium 6β-[Z-[2-(2-aminothiazol-4-yl)-3-(7-oxabicyclo[2.2.1]hept-2-yl)]propenamido]penicillanate

Z-[2-(2-Aminothiazol-4-yl)-3-(7-oxabicyclo[2.2.1]hept-2-yl)]propenoic acid (0.020g) was coupled to 6-aminopenicillanic acid (0.018g) as described in Example 1(d). Coupling proceeded for 2.25h; after saturating the aqueous phase with sodium chloride and acidifying to pH2 using 2M HCl, it was extracted with ethyl acetate: THF, 1:1 (2x). The combined organic extracts were washed with brine, then water was added and the pH adjusted to 6.5 by addition of aqueous $NaHCO_3$ with stirring. The aqueous phase was separated, a small further aqueous wash was added and the total aqueous phase was concentrated, then lyophilised to a powder (61mg). Chromatography of this material on HP20SS, eluting with acetone-water mixtures, afforded after pooling, concentration and lyophilisation of appropriate fractions the title penicillin (0.022g); $\nu_{max}$(KBr) 1766, 1655, 1610, and 1527cm$^{-1}$; δ($D_2O$, 250MHz) 1.20-2.05 (6H, m), 1.51, 1.61 (6H, 2m), 2.74 (1H, m), 4.23 (1H, s), 4.44, 4.73 (2H, 2m), 5.61 (2H, m), 6.13 (1H, 2d, both J = 11Hz), and 6.50 (1H, s); this product still had n.m.r. data consistent with very largely one ring isomer, probably endo; m/e 487 ($MH^+$) and 465 ($MH^+$ of free acid).

Example 7

Sodium 6β-[Z-[2-(2-aminothiazol-4-yl)-3-(exo-bicyclo[4.1.0]hept-7-yl)]propenamido]penicillanate

(a) Ethyl cis-bicyclo[4.1.0]heptane-7-carboxylate, exo and endo

This material was prepared according to a literature procedure (Org.Reactions , vol.18, ch.3, Reactions, using the first method cited). The exo-endo ratio was about 4:1 (n.m.r.). δ($CDCl_3$, 250MHz) 0.70-0.90 (2H, m), 1.00-1.35 (5H, m), 1.55-1.95 (4H, 2m), 3.43, and 3.78 (2H, 2m, ca . 4:1, J s 6.6 and 7.2Hz).

(b) 7-(Hydroxymethyl)bicyclo[4.1.0]heptane, exo and endo

Ethyl cis-bicyclo[4.1.0]heptane-7-carboxylate (3.24g) was reduced according to a literature procedure (W. Kirmse and K.H. Pook, Chem.Ber ., 1965, 98 , 4022), giving the title alcohol (2.07g).

(c) Bicyclo[4.1.0]heptane-7-carboxaldehyde. exo and endo

7-(Hydroxymethyl)bicyclo[4.1.0]heptane (2.07g) in anhydrous dichloromethane (4ml) was added in one portion to a stirred suspension of pyridinium chlorochromate (5.39g) in the same solvent (25ml). After 1.5h, when no alcohol was visible by t.l.c., workup was effected as in Example 1(a). Chromatography through a short pad of silica gel, eluting with ether, followed by evaporation afforded the aldehyde (1.40g); $\nu_{max}$(film) 2720, 1695, 1445, and 1410cm$^{-1}$; δ($CDCl_3$, 250MHz) 0.75-2.05 (11H, m), 9.01 (0.8H, d, J = 5.3Hz), and 9.66

(0.2H, m). [Reference as in part (b)].

(d) Methyl (E,Z)-[2-(2-acetamidothiazol-4-yl)-3-(exo-bicyclo[4.1.0]hept-7-yl)]propenoate

Bicyclo[4.1.0]heptane-7-carboxaldehyde (1.40g) and methyl 4-chloroacetoacetate (1.74g) in ethyl acetate (25ml) were cooled to 0°C and stirred while glacial acetic acid (0.66ml) and piperidine (1.15ml) were added. After 0.5h at the same temperature the solution was diluted with an equal volume of ethyl acetate and washed with water, 1M HCl, saturated aqueous $NaHCO_3$, water, and brine. After drying and evaporation an oil (3.56g) resulted; N -acetylthiourea (1.30g) was added and the mixture was heated in DMF (6ml) in the presence of 4A molecular sieves at 85-90°C for 1.5h. The reaction mixture was cooled, diluted with ether and washed with water (6x), then with brine, dried and evaporated to an orange oil (2.50g). Chromatogaphy on silica gel, eluting with ethyl acetate-hexane mixtures, gave firstly the Z-isomer (0.331g), m.p. 140-141°C (from ethyl acetate-hexane) (Found: C, 59.6; H, 6.3; N, 8.6; S, 9.9. $C_{16}H_{20}N_2O_3S$ requires C, 60.0; H, 6.25; N, 8.8; S, 10.0% ); $\nu_{max}$(KBr) 3072; 1709, 1658, 1603(w), and 1559cm$^{-1}$; $\delta$($CDCl_3$, 250MHz) 1.15-2.05 (10H, 3m), 2.12 (1H, dt, J =10.8 and 4.5Hz), 2.19 (3H, s), 3.87 (3H, s), 6.23 (1H, d, J =10.8Hz), 6.99 (1H, s), and 9.80 (1H, br s). Further elution gave the E-isomer (foam) (0.141g), $\delta$($CDCl_3$, 90MHz), inter alia , 6.42 (1H, d, J =10Hz) and 6.91 (1H, s).

(e) Z-[2-(2-aminothiazol-4-yl)-3-(exo-bicyclo[4.1.0]hept-7-yl)]propenoic acid

Methyl Z-[2-(2-acetamidothiazol-4-yl)-3-(exo-bicyclo[4.1.0]hept-7-yl)]propenoate (0.223g) was hydrolysed with 1M NaOH (4.0ml) as described in Example 1(c) with a reaction time of 5h. The solid obtained on acidification to pH3.7 with 5M HCl was filtered, washed with water and dried, giving the title acid (0.148g); $\nu_{max}$(KBr) 1611, 1559, and 1527cm$^{-1}$; $\delta$[$(CD_3)_2SO$, 250MHz] 1.00-2.00 (11H, 4m), 6.05 (1H, d, J=11Hz), 6.43 (1H, s), and 6.98 (2H, br s, $D_2O$ exch.); m/e 264 ($M^+$).

(f) Sodium 6$\beta$-[Z-[2-(2-aminothiazol-4-yl)-3-(exobicyclo[4.1.0]hept-7-yl)]propenamido]penicillanate

Z-[2-(2-Aminothiazol-4-yl)-3-(exo-bicyclo[4.1.0]hept-7-yl)]propenoic acid (0.132g) was coupled to 6-aminopenicillanic acid (0.119g) as described in Example 1(d). Coupling proceeded for 2h. Isolation by precipitation at pH 3.5 followed by chromatography of crude product (0.153g) as described in Example 4(f) afforded the title penicillin (0.072g); $\nu_{max}$(KBr) 1767, 1660(sh), 1609, and 1522cm$^{-1}$; $\delta$($D_2O$, 250MHz) 1.10-1.95 (11H, m), 1.49, 1.59 (6H, 2s), 4.19 (1H, s), 5.60 (2H, AB qt), 5.75 (1H, d, J =10.7Hz), and 6.35 (1H, s); m/e 485 ($MH^+$) and 463 ($MH^+$, free acid). Later eluting column fractions gave additional penicillin (0.056g) which was less pure by n.m.r. (considerable E-isomer).

Example 8

Sodium 6$\beta$-[Z-[2-(2-aminothiazol-4-yl)-3-(exo-bicyclo[3.1.0]hex-6-yl)]propenamido]penicillanate

(a) 6-(Hydroxymethyl)bicyclo[3.1.0]hexane, exo and endo

This material was prepared in two steps from cyclopentene according to a literature procedure (W. Kirmse and K.H. Pook, Chem.Ber., 1965, 98 , 4022), and twice distilled (Kugelrohr) to give the alcohol ; $\delta$-($CDCl_3$, 250MHz) 0.85-2.00 (10H, m, 9H on $D_2O$ exch.), 3.42 and 3.66 (2H, 2d, J =7.2 and 7.6Hz, ca . 3.5:1).

(b) Bicyclo[3.1.0]hexane-6-carboxaldehyde, exo and endo

6-(Hydroxymethyl)bicyclo[3.1.0]hexane (1.32g) was oxidised using pyridinium chlorochromate (4.00g) as described in Example 7(c). Workup as in Example 1(a) but eluting the column with ether afforded the

aldehyde (0.95g); $\nu_{max}$(film) 2860(m), 2720(w), and 1710cm$^{-1}$; $\delta$($CDCl_3$, 90MHz) 0.80-2.40 (9H, m), 9.06 and 9.43 (1H, 2d, J =5Hz and 7Hz, ca . 3.5:1) [Ref. as in part (a)].

(c) Methyl (E,Z)-[2-(2-acetamidothiazol-4-yl)-3-(exo-bicyclo[3.1.0]hex-6-yl)]propenoate

Bicyclo [3.1.0]hexane-6-carboxaldehyde (0.95g) was condensed with methyl 2-acetamidothiazol-4-acetate (1.85g) as described in Example 1(b), the reaction time being 17h. Chromatography of the crude product (2.80g) as described therein gave, firstly, the Z-isomer (0.229g); $\nu_{max}$(KBr) 1714, 1649, 1610(sh), and 1559cm$^{-1}$; $\delta$($CDCl_3$, 250MHz) 1.22 (1H, m), 1.50-2.25 (8H, m), 2.19 (3H, s), 3.86 (3H, s), 6.24 (1H, d, J =11Hz), 6.97 (1H, s), and 9.67 (1H, br s, $D_2O$ exch.); m/e 306($M^+$) (Found: M , 306.1033. $C_{15}H_{18}N_2O_2S$ requires M , 306.1038). Further elution of the column gave the E-isomer (0.466g); $\delta$($CDCl_3$, 90MHz), inter alia , 6.44 (1H, d, J =10Hz) and 6.98 (1H, s).

(d) Z-[2-(2-Aminothiazol-4-yl)-3-(exo-bicyclo[3.1.0]hex-6-yl)]propenoic acid

Methyl Z-[2-(2-acetamidothiazol-4-yl)-3-(exo-bicyclo[3.1.0]hex-6-yl)]propenoate (0.190g) was hydrolysed with 1M NaOH (3.1ml) as described in Example 1(c) (reaction time 4.5h). The solid obtained on acidification to pH4 was filtered, washed with ice-cold water, and dried to give the title acid (0.113g); $\nu_{max}$(KBr) 1640(sh), 1602, 1570(w), 1534, and 1448(w)cm$^{-1}$; $\delta$[$(CD_3)_2SO$, 250Mz] 0.95-1.30 (2H, m), 1.35-1.85 (6H, m), 1.92 (1H, approx. d, J =11Hz), 6.04 (1H, d, J =11Hz), 6.42 (1H, s), and 6.99 (2H, br s, $D_2O$ exch.); m/e 250($M^+$) (Found: M , 250.0783. $C_{12}H_{14}N_2O_2S$ requires M , 250.0776).

(e) Sodium 6β-[Z-[2-(2-aminothiazol-4-yl)-3-(exo-bicyclo[3.1.0]hex-6-yl)]propenamido]penicillanate

Z-[2-(2-Aminothiazol-4-yl)-3-(exo-bicyclo[3.1.0]hex-6-yl)]propenoic acid (0.105g) was coupled to 6-aminopenicillanic acid (0.100g) as described in Example 1(d), the coupling time being 2.75h. The reaction was filtered, washed with THF and water and the combined filtrate and washings evaporated to near dryness. The residue was dissolved in water containing saturated aqueous $NaHCO_3$ (1ml) and washed with ether:ethyl acetate, 1:1 (2x), backwashing each time with a little water. The total aqueous phase was acidified to pH2 and extracted with ethyl acetate (2x), then the combined organic extracts were washed with water (4x). Water was added, then the aqueous phase was basified to pH7 using aqueous $NaHCO_3$. After adding a little acetone to break an emulsion, the aqueous phase was separated and a further aqueous washing was added. The combined aqueous phases were concentrated to low volume, then lyophilised to give crude product (0.176g). Chromatography on HP20SS, eluting with acetone-water mixtures, followed by concentration and lyophilisation of appropriate fractions, afforded the penicillin (0.058g); $\nu_{max}$(KBr) 1767, 1655(sh), 1610, 1525, and 1510(sh)cm$^{-1}$; $\delta$($D_2O$, 250MHz) 1.16(2H, m), 1.35-1.90 (7H, m), 1.48, 1.59 (6H, 2s), 4.20 (1H, s), 5.59 (2H, ABqt), 5.77 (1H, d, J =10.5Hz), and 6.36 (1H, s); m/e 471 ($MH^+$) and 449($MH^+$, free acid). Later-eluting column fractions gave less pure penicillin (0.050g), including some E -isomer.

Example 9

Sodium 6β-[Z-[2-(2-aminothiazol-4-yl)-3-(endo-1-methoxybicyclo[2.2.2]oct-5-ene-2-yl)]propenamido]-penicillanate, isomers

(a) Exo,endo 1-methoxybicyclo[2.2.2]oct-5-ene-2-al

A solution of methyl 1-methoxybicyclo[2.2.2]oct-5-ene-2-carboxylate (5g) in ether (30ml) was added dropwise to a suspension of lithium aluminium hydride (2.2g) in ether at room temperature. The resulting suspension was stirred at room temperature for 1hr, after which t.l.c. showed complete consumption of the starting ester. The mixture was then treated dropwise with water (2.2ml), 15% w/v sodium hydroxide (2.2ml), water (6.6ml) and the resulting white solid filtered off. The solid was washed with ether and the combined filtrates evaporated under reduced pressure to give a yellow oil, which was vacuum distilled

(water pump) to give the intermediate alcohol as a colourless oil (3g). This was found to be a mixture of exo and eno isomers: δ($CDCl_3$) 0.5-0.65 and 0.77-0.91 (1H, m), 1.15-1.90 (5H, m), 1.95-2.20 (1H, m) 2.37-2.51 (1H, m), 3.05-3.29 (2H, m), 3.31-3.80 (4H, m), and 6.13-6.50 (2H, m).

The above alcohol (3g) was dissolved in dry dichloromethane (20ml) containing 4A molecular sieves. Pyridinium chlorochromate (6.3g) was added portionwise and the resulting mixture stirred at room temperature for 2.5hr. The brown solid was removed by filtration and washed with dichloromethane. The combined filtrate and washings were evaporated under reduced pressure to give a brown oil which was subjected to silica-gel chromatography using 5% ethyl acetate-hexane as eluant to facilitate the separation of the exo and endo isomers.

Exo isomer (0.35g)

δ($CDCl_3$) 1.20-1.75 (6H, m), 1.97-2.11 (1H, m), 2.50-2.75 (2H, m), 3.47 (3H, s), 6.23-6.5 (2H, m), and 9.90 (1H, d, J 1.9Hz).

Endo isomer (0.92g)

δ($CDCl_3$) 1.22-1.85 (6H, m), 2.62 (1H, bs), 2.70-2.84 (1H, m), 3.39 (3H, s) 6.23-6.40 (2H, m), and 9.50 (1H, d, J 3.3Hz).

(b) Methyl (E,Z)-[2-(2-N,N-dimethylaminomethyleneaminothiazol-4-yl)-3-(endo-1-methoxybicyclo[2.2.2]oct-5-ene-2-yl)]propenoate

A solution of diphenylamine (0.9g) in dry THF (20ml) at -30°C under argon was treated with 1.6M n-butyl lithium in hexane (3.16ml). After stirring at -30°C for 0.25hr the mixture was cooled to -70°C and a solution of methyl 2-(N,N-dimethylaminomethyleneamino)thiazol-4-yl acetate (1.2g) in dry THF (10ml) was added. After a further 0.25hr, the solution was treated with a solution of the endo-aldehyde (0.9g) (example 9a) in dry THF (5ml). After 0.25hr methanesulphonyl chloride (1.2ml) was added and the reaction mixture allowed to warm to room temperature. Ethyl acetate and water were then added, and the organic phase separated. After washing with water and saturated brine the organic phase was dried ($MgSO_4$) and evaporated under reduced pressure to give a brown oil which was flash chromatographed using ethyl acetate as eluant to remove the diphenylamine. The resulting oil was dissolved in dichloromethane (40ml) and treated with DBU (0.7ml). The reaction mixture was stirred at room temperature for 0.5hr, after which t.l.c. showed complete consumption of the starting material. The mixture was subjected to silica-gel column chromatography using ethyl acetate as eluant to give the Z-isomer (0.7g); $\nu_{max}$($CHCl_3$) 1720(sh), 1715, 1630(sh) and 1620cm$^{-1}$; δ($CDCl_3$) 1.15-1.30 (1H, m), 1.30-1.55 (2H, m), 1.60-1.85 (2H, m), 2.00-2.20 (1H, m), 2.47 (1H, bs), 3.08 and 3.11 (6H, 2s), 3.15-3.20 (1H, m), 3.27 (3H, s), 3.85 (3H, s), 6.19-6.40 (2H, m), 6.69 (1H, d, J 9.2Hz), 6.70(1H, s), and 8.19 (1H, bs).Further elution furnished the E-isomer (0.5g); $\nu_{max}$-($CHCl_3$) 1710, 1635(sh), and 1620cm$^{-1}$; δ($CDCl_3$) 1.10-1.15 (1H, m), 1.30-1.70 (4H, m), 1.80-2.00 (1H, m), 2.47 (1H, bs), 2.90-3.10 (1H, m) 3.08 and 3.10 (6H, 2s), 3.22 (3H, s), 3.72 (3H, s), 6.20-6.40 (2H, m), 6.79 (1H, d, J 10.9Hz), 6.81 (1H, s), and 8.28 (1H, bs).

(c) Methyl-Z-[2-(2-formamidothiazol-4-yl)-3-(endo-1-methoxybicyclo[2.2.2]oct-5-ene-2-yl)]propenoate

The Z -isomer of the amidine (Example 9b) (0.65g), was dissolved in acetic formic anhydride (20ml). The mixture was stirred at room temperature for 3hr then a further aliquot of acetic formic anhydride (10ml) was added. After stirring for a further 2hr the reaction mixture was evaporated under reduced pressure and the resulting oil redissolved in ethyl acetate. The organic phase was washed with water, saturated sodium bicarbonate solution, water, saturated brine and dried ($MgSO_4$). After removal of the solvent the resulting yellow oil was subjected to silica-gel column chromatography using 50% ethyl acetate-hexane as eluant to give the title compound (0.47g); $\nu_{max}$($CHCl_3$) 1780, 1720(sh), and 1710cm$^{-1}$; δ($CDCl_3$) 1.10-1.30 (1H, m), 1.30-1.60 (2H, m), 1.60-1.80 (2H, m), 2.05-2.15 (1H, m), 2.40-2.60 (1H, m), 3.25-3.45 (1H, s), 3.30 (3H, s), 3.86 (3H, s), 6.15-6.40 (2H, m), 6.58 (1H, d, J 9.9Hz), 6.55 (1H, s), 8.52 (1H, s), and 11.52 (1H, b s).

(d) Z-[2-(2-aminothiazol-4-yl)-3-(endo-1-methoxybicyclo[2.2.2]oct-5-ene-2-yl)]propenoic acid

A solution of the ester (Example 9c) (0.48g), in dry dioxan (7ml), was treated with a solution of sodium hydroxide (0.28g), in water (7ml). The resulting solution was heated under reflux for 1.5hr after which t.l.c. showed complete consumption of the starting ester. The mixture was neutralised (5N HCl) and the dioxan removed under reduced pressure. The aqueous phase was washed with ethyl acetate (2x). Ethyl acetate was added and the pH adjusted to 3 (5N HCl). The organic phase was separated and the aqueous phase washed with ethyl acetate (2x). The combined ethyl acetate phases were dried ($MgSO_4$) and their volume reduced to 10ml. Hexane was then added and the resulting white precipitate filtered and dried in vacuo to furnish the title acid (0.26g); $\nu_{max}$(KBr) 1700 and 1624cm$^{-1}$; $\delta$[$(CD_3)_2SO$] 0.93-1.10 (1H, m), 1.20-1.45 (2H, m), 1.55-1.80 (2H, m), 1.90-2.10 (1H, m), 2.35-2.55 (1H,m) 3.00-3.15 (1H, m),3.16 (3H,s), 6.16 (1H, d, J 8.7Hz), 6.28 (1H, d, J 10.5Hz), 6.33 (1H, dd, J 8.7Hz) 6.28 (1H, d, J 10.5Hz), 6.33 (1H, dd, J 8.7Hz), 6.35 (1H, s), 7.04 (2H, s), and 12.79 (1H, bs).

(e) Sodium 6$\beta$-[Z-[2-(2-aminothiazol-4-yl)-3-(endo-1-methoxybicyclo[2.2.2]oct-5-ene-2-yl)]propenamido]-penicillanate

Z-[2-(2-aminothiazol-4-yl)-3-(endo-1-methoxybicyclo[2.2.2]oct-5-ene-2-yl)]propenoic acid (0.1g), 1-hydroxybenzotriazole monohydrate (0.056g), and N ,N -dimethylaminopyridine (catalytic quantity) were stirred in dry DMF (3ml) whilst N ,N ′-dicyclohexylcarbodi-imide (0.071g) was added. The mixture was allowed to warm to room temperature and stirred under argon for 4hr. T.l.c. showed active ester to have formed. The mixture was filtered into a stirred solution of 6-aminopenicillanic acid (0.073g) and 1N sodium hydroxide (0.36ml) in water (2ml). The mixture was stirred at room temperature for 2hr when complete by t.l.c., water (5ml) was added and the pH of the mixture adjusted to 7.5. The resulting mixture was filtered and the filtrate washed with ethyl acetate. The aqueous phase was acidified to pH2 (1N HCl) and extracted twice with ethyl acetate. The combined organic phases were washed twice with water, and then after a further quantity of water had been added the pH was adjusted to 7.5 (1N NaOH). The aqueous phase was separated and the organic phase washed twice with water. The combined aqueous phases were evaporated to ca 5ml and subjected to HP20SS column chromatography eluting with aqueous THF mixtures. Appropriate fractions were combined, concentrated and freeze-dried to give the title penicillin (0.064g) as a 1:1 mixture of diastereoisomers; $\nu_{max}$(KBr) 1768, 1658, 1609, and 1526cm$^{-1}$; $\delta$($D_2O$) 1.05-1.20 (1H, m), 1.30-2.20 (12H, m), 2.53 (1H, bs), 2.93-3.18 (1H, m), 3.29 and 3.31 (3H, 2s), 4.23 (1H, s), 5.45-5.70 (2H, m), 6.00 and 6.04 (1H, 2d, J 11.4Hz), 6.10-6.25 (1H, m), and 6.35-6.55 (2H, m).

(f) Methyl (E,Z)-[2-(2-N,N-dimethylaminomethyleneaminothiazol-4-yl)-3-(exo-1-methoxybicyclo[2.2.2]oct-5-ene-2-yl)]propenoate

Exo-1-methoxybicyclo[2.2.2]oct-5-ene-2-al (Example 9a, (0.324g) was condensed with methyl [2-(N,N-dimethylaminomethyleneamino) thiazol-4-yl]acetate (0.43g) as described in Example 9(b). The workup and chromatography were carried out as described therein to furnish the title compounds , Z -isomer, (0.26g) (Found: M , 375.1616. $C_{19}H_{25}N_3O_3S$ requires M , 375.1617); $\nu_{max}$($CHCl_3$) 1720, 1630(sh), and 1620cm$^{-1}$; $\delta$-($CDCl_3$) 1.20-1.50 (3H, m), 1.55-1.70 (1H, m), 1.75-1.95 (1H, m), 1.95-2.20 (1H, m), 2.40-2.55 (1H, m), 2.85-3.05 (1H, m), 3.10 and 3.13 (6H, 2s), 3.29 (3H, s), 3.82 (3H, s), 6.24 (1H, d, J 8.8Hz), 6.44 (1H, d, J 8.8Hz), 6.72 (1H, s), 7.10 (1H, d, J 10.3Hz), and 8.21 (1H, bs). E-isomer, (0.09g), $\nu_{max}$($CHCl_3$) 1720 and 1620cm$^{-1}$; $\delta$($CDCl_3$) 1.20-1.55 (3H, m), 1.60-1.80 (2H, m), 1.85-2.10 (1H, m), 2.40-2.55 (1H, m), 2.75-2.90 (1H, m), 3.06 and 3.09 (6H, 2s), 3.20 (3H, s), 3.76 (3H, s), 6.20 (1H, d, J 8.8Hz), 6.38 (1H, d, J 8.8Hz), 6.79 (1H, s), 7.20 (1H, d, J 10.5Hz), and 8.24 (1H, s).

(g) Methyl-Z-[2-(2-formamidothiazol-4-yl)-3-(exo-1-methoxybicyclo[2.2.2]oct-5-ene-2-yl)]propenoate

Methyl-Z-[2-(2-N ,N -dimethylaminomethyleneaminothiazol-4-yl)-3-(exo-1-methoxybicyclo[2.2.2]oct-5-ene-2-yl)]propenoate (0.24g) was treated with acetic formic anhydride (20ml) as described in Example 9(c), to give the required ester (0.16g). (Found: M , 306.1039. $C_{15}H_{18}N_2O_3S$ requires M , 306.1038); $\nu_{max}$($CHCl_3$) 1780 and 1710cm$^{-1}$; $\delta$($CDCl_3$) 1.20-1.55 (3H, m), 1.55-1.75 (1H, m), 1.85-2.05 (1H, m), 2.52 (1H, bs), 2.95-3.15 (1H, m), 3.32 (3H, s), 3.83 (3H, s), 6.27 (1H, d, J 8.8Hz), 6.43 (1H, d, J 8.8Hz), 6.95 (1H, d, J 10.1Hz),

6.98 (1H, s), 8.58 (1H, s), and 11.23 (1H, bs).

(h) Z-[2-(2-aminothiazol-4-yl)-3-(exo-1-methoxybicyclo[2.2.2]oct-5-ene-2-yl)]propenoic acid

Methyl-Z-[2-(2-formamidothiazol-4-yl)-3-(exo-1-methoxybicyclo[2.2.2]oct-5-ene-2-yl)]propenoate (0.16g) was hydrolysed as described in Example 9(d) to give the title acid (0.08g); $\nu_{max}$(KBr) 3319, 3135, 3046, 1669, 1617, and 1559cm$^{-1}$; $\delta[(CD_3)_2SO]$ 1.00-1.48 (3H, m), 1.48-2.00 (3H, m), 2.46 (1H, bs), 2.73-2.95 (1H, m), 3.18 (3H, s), 6.25 (1H, d, J 8.8Hz), 6.40 (1H, s), 6.48 (1H, d, J 8.8Hz), 6.65 (1H, d, J 10.4Hz), 7.08 (1H, s), and 12.85 (1H, bs).

(i) Sodium 6$\beta$-[Z-[2-(2-aminothiazol-4-yl)-3-(exo-1-methoxybicyclo[2.2.2]oct-5-ene-2-yl)]propenamido]-penicillanate, isomers

The free acid from Example 9(h) (0.061g) was coupled to 6-aminopenicillanic acid (0.047g) using the method described in Example 9(e) to give the title penicillin (0.048g) as a freeze-dried solid. The N.M.R. spectrum indicated that the solid was a 1:1 mixture of diastereoisomers. $\nu_{max}$(KBr) 1769, 1654, and 1609cm$^{-1}$; $\delta(D_2O)$ 1.10-2.00 (13H, m), 2.52 (1H, bs), 2.65-2.90 (1H, m), 3.31 and 3.32 (3H, 2s), 4.20 (1H, s), 5.43-5.65 (2H, m), and 6.27-6.60 (3H, m).

## Example 10

Sodium 6$\beta$-[Z-[2-(2-aminothiazol-4-yl)-3-(cis-bicyclo[3.3.0]oct-2-yl)]propenamido]penicillanate

(a) Methyl cis-bicyclo[3.3.0]octan-2-al

cis-Bicyclo[3.3.0]octane-2-carboxylic acid (10g) was dissolved in DMF (200ml) and treated with potassium carbonate (5.4g) and methyl iodide (6ml). The mixture was stirred at room temperature overnight. Water and ethyl acetate were added, the organic phase was separated and the aqueous phase washed twice with ethyl acetate. The combined organic phases were washed with water, saturated brine and dried ($MgSO_4$). Evaporation under reduced pressure gave a yellow oil, which was purified by Kugelrohr distillation under reduced presure (mini-pump) to give a colourless liquid. This liquid was reduced with lithium aluminium hydride as described in example 9(a), and oxidised with pyridinium chlorochromate as described in Example 9(b), to give the required aldehyde (4.0g), purified by Kugelrohr distillation, as a colourless oil; $\delta$-($CDCl_3$) 0.90-1.81 (8H, m), 1.85-2.21 (2H, m), 2.18-2.40 (1H, m), 2.47-2.59 (1H, m), and 9.84 (1H, d, J 2.1Hz).

(b) Methyl (E,Z)-[2-(2-(N,N-dimethylaminomethyleneamino)thiazol-4-yl)-3-(cis-bicyclo[3.3.0]oct-2-yl)-]propenoate

Methyl cis-bicyclo[3.3.0]octan-2-al (2.0g) was condensed with methyl 2-(N ,N - dimethylaminomethyleneamino)thiazol-4-yl acetate (2.5g) as described in Example 9(b) to give the required acrylates : Z-isomer (1.15g) as a 1:4 mixture of diasteroisomers; (Found: M , 347.1678. $C_{18}H_{25}N_3O_2S$ requires M , 347.1667); $\nu_{max}$($CHCl_3$) 1720, 1700(sh), 1635, and 1620cm$^{-1}$; $\delta(CDCl_3)$ 0.90-1.74 (8H, m), 1.75-2.10 (2H, m), 2.15-2.35 (1H, m), 2.40-2.60 (2H, m), 3.09 and 3.11 (6H, 2s), 3.85 (3H, s), 6.68 and 6.69 (1H, 2s), 6.84 and 6.91 (1H, 2d, J 10Hz), and 8.14 (1H, s).

E-isomer (1.16g) as a 1:4 mixture of diastereoisomers; $\delta(CDCl_3)$ 1.00-1.85 (8H, m), 1.83-2.05 (2H, m), 2.15-2.35 (1H, m), 2.35-2.65 (2H, m), 3.06 and 3.09 (6H, 2s), 3.75 (3H, s), 6.64 and 6.66 (1H, 2s), 6.98 and 7.09 (1H, 2d, J 10.2Hz), 8.25, and 8.28 (1H, 2s).

(c) Methyl-Z-[2-(2-formamidothiazol-4-yl)-3-(cis-bicyclo[3.3.0]oct-2-yl)]propenoate

Methyl-Z-[2-(2-N ,N -dimethylaminomethyleneamino)thiazol-4-yl)-3-(cis-bicyclo[3.3.0]oct-2-yl)-]propenoate (0.97g) was treated with acetic formic anhydride as described in Example 9(c), to give the title compound as a colourless oil (0.57g). (Found: M , 320.1200. $C_{16}H_{20}N_2O_3S$ requires M , 320.1195); $\nu_{max}$-($CHCl_3$) 1720(sh) and 1710cm$^{-1}$; δ($CDCl_3$) 1.00-1.30 (7H, m), 1.80-2.08 (2H, m), 2.05-2.35 (1H, m), 2.35-3.15 (3H, m), 3.85 (3H, s), 6.57 and 6.69 (1H, 2d, J 10Hz), 6.97 and 6.99 (1H, 2s), 8.50 (1H, s), and 11.23 (1H, bs).

(d) Z-[2-(2-aminothiazol-4-yl)-3-(cis-bicyclo[3.3.0]oct-2-yl)]propenoic acid

Methyl-Z-[2-(2-formamidothiazol-4-yl)-3-(cis-bicyclo[3.3.0]oct-2-yl)]propenoate (0.50g) was hydrolysed as described in Example 9(d), to give the title acid (0.3g) as a white solid; $\nu_{max}$(KBr) 1628, 1559, and 1528cm$^{-1}$; δ[$(CD_3)_2SO$] 1.00-1.60 (7H, m), 1.62-2.20 (3H, m), 2.25-2.95 (3H, m), 6.32 and 6.47 (1H, 2d, J 10Hz), 6.36 (1H, s), 7.03 (2H, s), and 12.84 (1H, bs).

(e) Sodium 6β-[Z-[2-(2-aminothiazol-4-yl)-3-(cis-bicyclo[3.3.0]oct-2-yl)]propenamido]penicillanate

The acid from Example 10(d) (0.06g) was coupled to 6-aminopenicillanic acid (0.047g) using the method described in Example 9(e) to give the title penicillin (0.052g) as a freeze-dried solid; $\nu_{max}$(KBr) 1766, 1609, and 1525cm$^{-1}$; δ($D_2O$) 1.12-2.89 (19H, m), 6.92 (1H, s), 5.64 and 5.67 (1H, 2d, J 3.9Hz), 5.72 and 5.73 (1H, 2d, J 3.9Hz), 6.34 and 6.36 (1H, 2d, J 10.3Hz), 6.53 and 6.54 (1H, 2s).

Example 11

Sodium 6β-[Z-[2-(2-aminothiazol-4-yl)-3-(exo-bicyclo[2.2.2]oct-5-ene-2-yl)]propenamido]penicillanate

(a) Methyl (E,Z)-[2-(2-(N,N-dimethylaminomethyleneamino)thiazol-4-yl)-3-(exo-bicyclo[2.2.2]oct-5-ene-2-yl)-]propenoate

Exo-bicyclo[2.2.2]oct-5-ene-2-al (1.190g), prepared as described by Diels et al (Annalen , 1930, 478) was condensed with methyl 2-(N ,N -dimethylaminomethyleneamino)thiazol-4-yl acetate as described in Example 9(b) to give the title acrylates , the Z-isomer (1.30g), being a white crystalline solid. (Found: C, 62.59; H, 6.76; N, 12.16; S, 9.28%. $C_{18}H_{23}N_3O_2S$ requires C, 62.58; H, 6.71; N, 12.16; S, 9.28%); $\nu_{max}$-($CHCl_3$) 1715, 1630, and 1620cm$^{-1}$; δ($CDCl_3$) 1.00-1.40 (3H, m), 1.43-1.73 (2H, m), 1.80-2.00 (1H, m), 2.43-2.63 (2H, m), 2.80-2.97 (1H, m), 3.09 and 3.13 (6H, 2s), 3.86 (3H, s), 6.24 (1H, dd, J 7.5Hz), 6.36 (1H, dd, J 7.5Hz), 6.56 (1H, d, J 10.5Hz), 6.68 (1H, s), and 8.25 (1H, s). The E-isomer (0.90g) was a colourless oil; $\nu_{max}$ ($CHCl_3$) 1710 and 1620cm$^{-1}$; δ($CDCl_3$) 1.00-1.50 (6H, m), 1.65-1.88 (1H, m), 2.40-2.60 (2H, m), 2.66-2.83 (1H, m), 3.11 and 3.14 (6H, 2s), 3.74 (3H, s), 6.24 (1H, dd, J 7.5Hz), 6.39 (1H, dd, J 7.5Hz), 6.64 (1H, s), 6.75 (1H, d, J 10.6Hz), and 8.50 (1H, s).

(b) Methyl Z-[2-(2-formamidothiazol-4-yl)-3-(exo-bicyclo[2.2.2]oct-5-ene-2-yl)]propenoate

Methyl Z-[2-(2-(N ,N -dimethylaminomethyleneamino)thiazol-4-yl)-3-(exo-bicyclo[2.2.2]oct-5-ene-2-yl)-]propenoate (0.87g) was treated with acetic formic anhydride as described in Example 9(c) to give the title compound (0.56g) as a white crystalline solid (Found: C, 60.43; H, 5.71; N, 8.82; S, 10.25. $C_{16}H_{18}N_2O_3S$ requires C, 60.36; H, 5.70; N, 8.80; S, 10.07%); $\nu_{max}$($CHCl_3$) 1705 and 1543cm$^{-1}$; δ($CDCl_3$) 1.00-2.07 (6H, m), 2.40-2.60 (2H, m), 2.90-3.10 (1H, m), 3.87 (3H, s), 6.20 (1H, dd, J 7.5Hz), 6.32-6.48 (2H, m), 6.96 (1H, s), and 8.51 (1H, s).

(c) Z-[2-(2-aminothiazol-4-yl)-3-(exo-bicyclo[2.2.2]oct- 5-ene-2-yl)]propenoic acid

Methyl Z-[2-(2-formamidothiazol-4-yl)-3-(exo-bicyclo[2.2.2]oct-5-ene-2-yl)]propenoate (0.25g) was

hydrolysed as described in Example 9(d) to furnish the title acid as a white solid (0.13g); $\nu_{max}$(KBr) 1629 and 1559cm$^{-1}$; δ[$(CD_3)_2SO$] 0.78-1.60 (5H, m), 0.71-0.90 (1H, m), 2.30-2.50 (1H, m), 2.65-2.89 (1H, m), 6.00-6.49 (4H, m), 6.98 (2H, s), and 11.00-12.00 (1H, bs); addition of $D_2O$ caused the peaks at 6.98 (2H, s) and 11.00-12.10 (1H, bs) to disappear.

(d) Sodium 6β-[Z-(2-[2-aminothiazol-4-yl)-3-(exo-bicyclo[2.2.2]oct-5-ene-2-yl)]propenamido]penicillanate

The acid from Example 11(b) (0.06g) was condensed with 6-aminopenicillanic acid (0.052g) using the method described in Example 9(e) to give the title penicillin (0.042g) as a white freeze-dried solid, a 1:1 mixture of diastereoisomers; $\nu_{max}$(KBr) 1764, 1610, and 1527cm$^{-1}$; δ($D_2O$) 1.50-1.88 (1H, m), 1.20-1.40 (3H, m), 1.50-1.80 (1H, m), 1.61 and 1.62 (3H, 2s), 1.68 and 1.71 (3H, 2s), 1.86-2.08 (1H, m), 2.45-2.83 (3H, m), 4.33 and 4.34 (1H, 2s), 5.68 (1H, d, J 4.1Hz), 5.73 and 5.74 (1H, 2d, J 4.1Hz), 6.06 and 6.08 (1H, 2d, J 10.9Hz), 6.30-6.40 (1H, m), and 6.48-6.60 (2H, m).

Example 12

Exo-and endo- sodium 6β-[Z-[2-(2-aminothiazol-4-yl)-3-(3-oxabicyclo[3.1.0]hex-6-yl)]propenamido]-penicillanates

(a) Exo- and endo- ethyl 3-oxabicyclo[3.1.0]hexane-6-carboxylate

Ethyl diazoacetate (3.42g) was diluted with ether (3.5ml) and added dropwise to a stirred mixture of rhodium acetate dimer (0.25g) in 2,5-dihydrofuran (3.5g) at 0°C. Vigorous effervescence occurred; when addition was complete, the mixture was allowed to regain ambient temperature and stirred for 2h. The mixture was filtered through Celite, the precipitate was washed with ether and the combined filtrate and washings were washed twice with water, once with brine, dried and evaporated to an orange oil. Kugelrohr distillation afforded the title ester (2.19g), which by n.m.r. analysis was contaminated with some diethyl maleate and fumarate; δ($CDCl_3$, 90MHz), inter alia , 1.10-2.20 (6H, m) and 3.60-4.40 (6H, m); m/e (chemical ionisation, $NH_3$), 157 ($MH^+$, 42%).

(b) Exo- and endo-6-(hydroxymethyl)-3-oxabicyclo[3.1.0]hexane

Exo /endo - ethyl 3-oxabicyclo[3.1.0]hexane-6-carboxylate (2.13g) was reduced using lithium aluminium hydride (0.73g) as described in Example 9(a). In this case the products were too water-soluble for aqueous washing, so the final ether solution was dried and evaporated to near dryness, then the residue was distilled (Kugelrohr) to afford the alcohols (0.72g); $\nu_{max}$(film) 3400(br) and 1650(m)cm$^{-1}$; δ($CDCl_3$, 90MHz) 0.90-1.90 (3H, m), 2.41 (1H, brs), and 3.30-3.95 (6H, m); m/e (chemical ionisation, $NH_3$), 132 ($MH_4^+$, 62%) and 115 ($MH^+$, base peak).

(c) Exo- and endo-3-oxabicyclo[3.1.0]hexane-6-carboxaldehyde

Exo /endo -6-(hydroxymethyl)-3-oxabicyclo[3.1.0] hexane (0.72g) was oxidised using pyridinium chlorochromate as described in Example 1(a). The title aldehydes (0.44g) showed $\nu_{max}$(film) 2750(m), 1770-(w), and 1700(vs)cm$^{-1}$; δ($CDCl_3$, 90MHz), 1.50-2.40 (3H, m), 3.65-4.30 (4H, m), 9.36, and 9.51 (1H, 2d, J s=5Hz and 6Hz, 3:1); m/e (chem.ionis., $NH_3$) 130 ($MNH_4^+$, base peak).

(d) Exo- and endo- methyl E,Z-[2-(2-acetamidothiazol-4-yl)-3-(3-oxabicyclo[3.1.0]hex-6-yl)]propenoate

Exo /endo -3-oxabicyclo[3.1.0]hexane-6-carboxaldehyde (0.44g) was converted in two steps to the aminothiazole as described in Example 7(d). Chromatography on silica gel, eluting with ethyl acetate-hexane mixtures, gave (after two columns) a complete separation of the exo /endo -isomers; the Z, exo-

ester (0.13g) had m.p. 206-211°C (prel.soft., from ethyl acetate-hexane). (Found: C, 53.5; H, 5.1; N, 9.2; S, 10.7; M , 308.0847. $C_{14}N_{16}N_2O_4S$ requires C, 54.5; H, 5.2; N, 9.1; S, 10.4%; M , 308.0831); $\nu_{max}$(KBr) 1717, 1654, 1554, and 1434cm$^{-1}$; δ($CDCl_3$, 250MHz) 1.86 (2H, s), 2.18 (1H, m), 2.23 (3H, s), 3.78, 3.98 (4H, approx.dd, J =8.3Hz), 3.87 (3H, s), 6.30 (1H, d, J =11Hz), 7.02 (1H, s), and 9.19 (1H, brs, $D_2O$ exch). The Z-, endo- ester (0.060g) showed δ($CDCl_3$, 250MHz), inter alia , 2.12 (2H, d, J =7.8Hz), 6.91 (1H, d, J =10.2Hz), and 6.93 (1H, s). Further elution of the first column afforded the E-ester (exo /endo -), δ($CDCl_3$, 90MHz), inter alia , 1.85 (3H, brs), 6.49 (0.7H, d, J =10Hz), 7.02, 7.05 (1H, 2s), and 7.23 (0.3H, d, J =7Hz).

(e) Z-[2-(2-Aminothiazol-4-yl)-3-(exo-3-oxabicyclo[3.1.0]hex-6-yl)]propenoic acid

Methyl Z-[2-(2-acetamidothiazol-4-yl)-3-(exo-3-oxabicyclo[3.1.0]hex-6-yl)]propenoate (0.092g) was hydrolysed and the crude product isolated as described in Example 6(c), extracting with THF (4x). Chromatography as described therein afforded, on pooling and evaporation of appropriate fractions, the title acid (0.065g); $\nu_{max}$(KBr) 1620(sh), 1602, 1575, 1560(sh), and 1535cm$^{-1}$; δ[$(CD_3)_2SO$, 250MHz)] 1.86 (2H, brs), 1.90-2.10 (1H, m), 3.63, 3.80 (4H, approx. dd, J =8.5Hz), 6.04 (1H, d, J =11Hz), 6.46 (1H, s), and 6.98 (2H, brs, $D_2O$ exch.); m/e 252 ($M^+$). (Found: M , 252.0567. $C_{11}H_{12}N_2O_3S$ requires M , 252.0569).

(f) Sodium 6β-[Z-(2-(2-aminothiazol-4-yl)-3-(exo-3-oxabicyclo[3.1.0]hex-6-yl)]propenamido]penicillanate

Z-[2-(2-Aminothiazol-4-yl)-3-(exo-3-oxabicyclo[3.1.0]hex-6-yl)]propenoic acid (0.059g) was coupled to 6-aminopenicillanic acid (0.056g) and purified by HP20SS chromatography as described in Example 6(d). The title penicillin (0.035g) showed $\nu_{max}$(KBr) 1764, 1650(sh), 1609, 1527, and 1458(w)cm$^{-1}$; δ($D_2O$, 250MHz) 1.49, 1.59 (6H, 2s), 1.50-1.65 (1H, m), 1.95 (2H, brs), 3.75, 3.94 (4H, 2m), 4.21 (1H, s), 5.60 (2H, brs) 5.85 (1H, d, J =10.8Hz), and 6.44 (1H, s); m/e 495 ($MNa^+$), 473 ($MH^+$), and 451 ($MH^+$, free acid). Further elution of the column afforded less pure penicillin (0.030g).

(g) Z-[2-(2-Aminothiazol-4-yl)-3-(endo-3-oxabicyclo[3.1.0]hex-6-yl)]propenoic acid

Methyl Z-[2-(2-acetamidothiazol-4-yl)-3-(endo-3-oxabicyclo[3.1.0]hex-6-yl)]propenoate (0.054g) was hydrolysed as in Example 6(c). Chromatography as described therein followed by pooling and evaporation of appropriate fractions afforded the title acid (0.027g); δ[$(CD_3)_2SO$, 250MHz)] 1.95-2.20 (3H, m), 3.80 (4H, ABqt, J =8.7Hz), 6.32 (1H, d, J =10Hz), 6.37 (1H, s), and 7.02 (2H, brs, $D_2O$ exch.).

(h) Sodium 6β-[Z-[2-(2-aminothiazol-4-yl)-3-(endo-3-oxabicyclo[3.1.0]hex-6-yl)]propenamido]penicillanate

Z-[2-(2-Aminothiazol-4-yl)-3-(endo-3-oxabicyclo[3.1.0]hex-6-yl)]propenoic acid (0.026g) was coupled to 6-aminopenicillanic acid (0.025g) and purified by HP2OSS chromatography as described in Example 6(d). The title penicillin (0.029g) showed $\nu_{max}$(KBr) 1766, 1660(sh), 1611, 1527, 1458(w), and 1399cm$^{-1}$; δ($D_2O$, 250MHz) 1.50, 1.59 (6H, 2s), 1.89 (1H, m), 2.05-2.20 (2H, m), 3.94 (4H, s), 4.22 (1H, s), 5.62 (2H, s), 6.23 (1H, d, J =9.5Hz), and 6.48 (1H, s); m/e 495 ($MNa^+$), 473 ($MH^+$), and 451 ($MH^+$, free acid).

Example 13

Exo- and endo- sodium 6β-[2-(2-aminothiazol-4-yl)-3-(bicyclo[4.1.0]hept-3-ene-7yl)]propenamido]-penicillanate

(a) Exo- and endo- ethyl bicyclo[4.1.0]hept-3-ene-7-carboxylate

Cyclohexa-1,4-diene (5.1g) was allowed to react with ethyl diazoacetate (4.56g) diluted with pentane (10ml) as described in Example 12(a). Distillation afforded the title ester (4.18g) containing traces of diethyl fumarate and maleate by n.m.r.; $\nu_{max}$(film) 3030 and 1720cm$^{-1}$; δ($CDCl_3$, 90MHz) 1.25 (3H, 2t), 1.10-1.50

(1H, m), 1.67 (2H, brs), 2.36 (4H, brs), 3.94-4.36 (2H, 2m), 5.45, and 5.52 (2H, 2brs); m/e 166($M^+$, 87%).

(b) Exo- and endo-7-(hydroxymethyl)bicyclo[4.1.0]hept-3-ene

Exo - and endo - ethyl bicyclo[4.1.0]hept-3-ene-7-carboxylate (4.30g) was reduced using lithium aluminium hydride (1.39g) as described in Example 9(a). Distillation gave the mixture of alcohols (2.30g); $\nu_{max}$ 3700-3050(br) and 1655 (m-w)$cm^{-1}$; δ($CDCl_3$, 250MHz) 0.92, 1.05-1.20 (3H, s+m), 1.39 (1H, s, $D_2O$ exch.), 2.05-2.50 (4H, m), 3.52, 3.64 (2H, 2d, J s=7.1 and 6.9Hz, ca 3:1), 5.47, and 5.61 (2H, 2m, ca 3:1); m/e 124 ($M^+$, 10%) (Found: M , 124.0889. $C_8H_{12}O$ requires M , 124.0888).

(c) Exo- and endo- bicyclo[4.1.0]hept-3-ene-7-carboxaldehyde

Exo /endo -7-hydroxymethylbicyclo[4.1.0]hept-3-ene (2.30g) was oxidised using pyridinium chlorochromate (6.00g) as described in Example 1(a). Chromatography afforded the aldehydes (1.73g); $\nu_{max}$(film) 3025, 2720, 1700, and 1650(sh)$cm^{-1}$; δ($CDCl_3$, 90MHz) 1.70-2.10 (3H, m), 2.10-2.70 (4H, m), 5.48, 5.74 (2H, 2m, ca 2.5:1), 9.21, and 9.35 (1H, 2m, d, J =4Hz + dd, J s=5Hz and 2Hz); m/e 122($M^+$, 100%) (Found: M , 122.0734. $C_8H_{10}O$ requires M , 122.0732).

(d) Exo- and endo- methyl E,Z-[2-(2-acetamidothiazol-4-yl)-3-(bicyclo[4.1.0]hept-3-ene-7-yl)]propenoate

Exo /endo -bicyclo[4.1.0]hept-3-ene-7-carboxaldehyde (1.04g) was condensed with methyl 2-acetamidothiazol-4-acetate (1.71g) as described in Example 1(b). Chromatography (ethyl acetate-hexane mixtures, silica) afforded the solid Z-exo-ester (0.15g); δ($CDCl_3$, 250MHz) 1.33 (2H, brs), 1.95-2.55 (5H, m), 2.19 (3H, s), 3.85 (3H, s), 5.51 (2H, brs), 6.35 (1H, d, J =11Hz), 6.99 (1H, s), and 9.82 (1H, brs, $D_2O$ exch.); m/e 318 ($M^+$). Further elution gave, firstly, a compound (0.25g) as a foam, δ($CDCl_3$, 250MHz), inter alia 2.06 (3H, s), 6.79 (1H, t, J =7.8Hz) and 6.95 (1H, s); m/e 378 (100%); the cyclopropane ring appeared to have opened with addition of the elements of acetic acid; secondly the E-esters (exo /endo ) (0.66g); δ($CDCl_3$, 90MHz), inter alia, 6.58, 7.07 (1H, 2d), 6.91, and 6.97 (1H, 2s).

Alternatively, conversion of the above aldehyde (0.64g) into the aminothiazole by the two-step procedure of Example 7(d) gave on chromatography the title Z-ester (exo :endo , ca 1:3) as a white solid (0.14g); m.p. 170-174°C (from ethyl acetate-hexane) (Found: C, 60.1; H, 5.7; N, 8.7; S, 10.0. $C_{16}H_{18}N_2O_3S$ requires C, 60.4; H, 5.7; N, 8.8; S, 10.1%); $\nu_{max}$(KBr) 1726, 1700, 1659, 1613, and 1554$cm^{-1}$; δ($CDCl_3$, 250MHz), inter alia (endo ) 1.58 (2H, approx. dd), 2.11 (3H, s), 5.41 (2H, brs), 6.68 (1H, d, J =10.8Hz), and 6.94 (1H, s). The exo /endo assignments were confirmed by nucleur Overhauser measurements at 400HMz on the recrystallized product (exo :endo was then ca 2:9).

(e) Exo- and endo-Z-[2-(2-aminothiazol-4-yl)-3-(bicyclo[4.1.0]hept-3-ene-7-yl)]propenoic acid

Combined methyl exo - and endo -Z-[2-(2-acetamidothiazol-4-yl)- 3-(bicyclo[4.1.0]hept-3-ene-7-yl)-]propenoate (0.220g) was hydrolysed as in Example 1(c) using 1MNaOH (5.2ml). Extraction from aqueous solution at pH2 with ethyl acetate (2x) then once with ethyl acetate:THF, 1:1 followed by drying and evaporation afforded crude product (0.164g). Chromatography as in Example 6(c) afforded firstly the title exo-acid (0.072g); $\nu_{max}$(KBr) 1660(sh), 1602, 1575(sh), 1533, 1427, and 1394$cm^{-1}$; δ[$(CD_3)_2SO$, 250MHz] 1.28 (2H, s), 2.06 (1H, dt, J s=11Hz and 4.2Hz), 2.15-2.50 (4H, m), 5.50 (2H, s), 6.16 (1H, d, J =11Hz), 6.42 (1H, s), 6.98 (2H, brs, $D_2O$ exch.), and 12.75 (1H, brs, $D_2O$ exch.); m/e 262 ($M^+$, 100%) (Found: M , 262.0773. $C_{13}H_{14}N_2O_2S$ requires M , 262.0776). Further elution gave very largely the endo-acid (0.036g); δ-(do.), inter alia , 1.35-1.50 (2H, m), 5.63 (2H, s), 6.09 (1H,d, J =10Hz), and 6.96 (2H, brs, $D_2O$ exch.); about 20% of the exo -acid was also present.

(f) Sodium 6β-[Z-[2-(2-aminothiazol-4-yl)-3-(exo- bicyclo[4.1.0]hept-3-ene-7-yl)]propenamido]penicillanate

Exo -Z -[2-(2-aminothiazol-4-yl)-3-(bicyclo[4.1.0]hept-3-ene-7-yl)]propenoic acid (0.065g) was coupled to 6-aminopenicillanic acid (0.059g) and purified as described in Example 6(d) using HP20SS. The title

penicillin (0.059g) showed $\nu_{max}$(KBr) 1766, 1650(sh), 1609, and 1522cm$^{-1}$; δ($D_2O$, 250MHz) 1.33 (2H, brs), 1.48, 1.59 (6H, 2s), 1.68 (1H, dt, J s=10.8Hz and 4.2Hz), 2.36 (4H, brs), 4.19 (1H, s), 5.52 (2H, brs), 5.55 (2H, ABqt), 5.90 (1H, d, J =10.8Hz), and 6.37 (1H, s); m/e 505 ($MNa^+$), 483 ($MH^+$), and 461 ($MH^+$, free acid).

(g) Sodium 6β-[Z-[2-(2-aminothiazol-4-yl)-3-(endo- bicyclo[4.1.0]hept-3-ene-7-yl)]propenamido]penicillanate

In the same manner as part (f) the endo -acid (0.034g) was coupled to 6-aminopenicillanic acid (0.031g). After purification the penicillin (0.033g) was obtained; $\nu_{max}$(KBr) 1762, 1650, 1617, and 1522cm$^{-1}$; δ($D_2O$, 250MHz) 1.10-1.40 (2H, m), 1.49, 1.59 (6H, 2s), 1.75-1.90 (1H, m), 2.00-2.50 (4H, m), 4.22 (1H, s), 5.61 (2H, ABqt), 5.69 (2H, s), 6.13 (1H, d, J =10.4Hz), and 6.39 (1H, s); m/e 505 ($MNa^+$, weak), 483 ($MH^+$), and 461 ($MH^+$, free acid). The n.m.r. spectrum also showed about 15% of the exo -isomer.

Example 14

Sodium 6β-[Z-[2-(2-aminothiazol-4-yl)-3-(exo-bicyclo[5.1.0]oct-8-yl)]propenamido]penicillanate

a) Exo- and endo- ethyl bicyclo[5.1.0]octane-8-carboxylate

Ethyl diazoacetate (8.89g) was diluted with hexane (6ml) and added dropwise to a stirred suspension of anhydrous copper sulphate (0.060g) in hexane (30ml) and cycloheptene (18.2ml) at 85°C. Vigorous effervescence occured; when addition was complete heating was continued for a further 1.5 hours. The mixture was coded, filtered through Celite and the precipitate washed with hexane. The filtrate was evaporated to near dryness and distilled to give the title ester (8.02g), boiling point 110°C at 2.5mm Hg; $\nu_{max}$ (film) 2970 (m) and 1620(s)cm$^{-1}$; δ($CDCl_3$, 90MHz) 3.95-3.40 (2H, m), and 0.50-2.30 (16H, m), also some signals due to diethyl maleate and fumarate.

b) Exo- and endo- 8-(hydroxymethyl)bicyclo[5.1.0]octane

Exo - and endo -ethyl bicyclo[5.1.0]octane-8-carboxylate (5.26g) was reduced using lithium aluminium hydride (1.38g) as described in Example 9(a). Kugelrohr distillation at an oven temperature of 180°C and at water pump pressure gave the title alcohol (2.65g) as a mixture of exo - and endo - isomers; $\nu_{max}$ ($CHCl_3$) 3620(w) and 3020(s)cm$^{-1}$; δ($CDCl_3$, 90MHz), inter alia 1.52 (1H, s, $D_2O$ exch.), 3.40, 3.75 (2H, 2d, J =8Hz, ca . 3:1); m/e 140 ($M^+$, 5%).

c) Exo- and endo- bicyclo[5.1.0]octane-8-carboxaldehyde

Exo - and endo -8-(hydroxymethyl)bicyclo[5.1.0]octane (2g) was oxidised using pyridinium chlorochromate (4.61g) as described in Example 1(a). Chromatography on silica gel using ether-pentane mixtures gave the desired aldehyde (1.84g) as a mixture of exo -and endo - isomers; $\nu_{max}$ ($CHCl_3$) 3010(m) and 1680(s)cm$^{-1}$; δ (90MHz, $CDCl_3$) 0.5-2.0 (13H, m), 9.05, and 9.45 (1H, 2d, J s=5Hz, ca 4:1); m/e 137 ($M-H^+$, 15%).

d) Methyl E,Z-[2-(2-acetamidothiazol-4-yl)-3-(exo-bicyclo[5.1.0]oct-8-yl)]propenoate

Exo - and endo - bicyclo [5.1.0]octane-8-carboxaldehyde (1.4g) was condensed with methyl 2-acetamidothiazol-4-acetate (2.23g) as described in Example 1(b). Chromatography on silica gel using ethyl acetate-hexane mixtures gave a yellow oil from which solid Z-exo-ester (0.30g) was crystallised using an ether-hexane mixture; $\nu_{max}$ (Nujol) 3180(w), 1700(s), and 1560(m)cm$^{-1}$; δ(250MHz, $CDCl_3$) 1.0-1.5 (7H, m), 1.75 (3H, m), 2.20 (7H, m), 3.85 (3H, s), 6.22 (1H, d, J =11.0Hz), 7.0 (1H, s), and 10.40 (1H, brs, $D_2O$ exch.); m/e 334 ($M^+$, 42%); m.p. 123-124°C (from ethyl acetate-hexane) (Found: C, 61.2; H, 6.4; N, 8.4; S,

9.5. $C_{15}H_{22}N_2O_3S$ requires C, 61.1; H, 6.6; N, 8.4; S, 9.6%). Further elution gave the E-exo-ester (1.08g); δ (90MHz, $CDCl_3$), inter alia 6.47 (1H, d, J =10Hz) and 6.95 (1H, s).

e) Z-[2-(2-aminothiazol-4-yl)-3-(exo-bicyclo[5.1.0]oct-8-yl)]propenoic acid

Methyl Z-[2-(2-acetamidothiazol-4-yl)-3-(exo -bicyclo[5.1.0]oct-8-yl)]propenoate (0.25g) was hydrolysed as described in Example 1(c) using 1M NaOH (5.6ml) to give the title Z-exo-acid (0.171g); $\nu_{max}$ (KBr) 2916(s), 1642(m), 1597(s), and 1532(s)$cm^{-1}$; δ [250MHz, $(CD_3)_2SO$] 0.9-1.9 (11H, m), 1.92 (1H, d, J =10.9Hz), 6.05 (1H, d, J =10.9Hz), 6.42 (1H, s), 6.95 (2H, s, $D_2O$ exch.), and 12.25 (1H, brs, $D_2O$ exch.); m/e (FAB) 279 ($MH^+$, 100%) [Found: $M-H_2O^+$, 260.0998. $C_{14}H_{16}N_2OS$ requires $M-H_2O^+$, 260.0983].

f) Sodium 6β-[Z-[2-(2-aminothiazol-4-yl)-3-(exo-bicyclo[5.1.0]oct-8-yl)]propenamido]penicillanate

Z-[2-(2-aminothiazol-4-yl)-3-(exo -bicyclo[5.1.0]oct-8-yl)]propenoic acid (0.160g) was coupled to 6-aminopenicillanic acid (0.137g) and purified as described in Example 6(d) using HP20SS. The title penicillin (0.075g) showed $\nu_{max}$ (KBr) 1766(s), 1650(sh), 1609(s), and 1522(m)$cm^{-1}$; δ (250MHz, $D_2O$) 1.0-1.5 (17H, m), 1.52 (3H, s), 1.65 (3H, s), 2.15 (3H, m), 4.25 (1H, s), 5.65 (2H, dd), 5.85 (1H, d, J =10.8Hz), and 6.40 (1H, s); m/e (FAB) 499 ($MH^+$, 25%).

## Example 15

Sodium 6β-[Z-[2-(2-aminothiazol-4-yl)-3-(exo-1-methylbicyclo[3.1.0]hex-6-yl)]propenamido]penicillanate, diastereoisomers.

(a) Exo- and endo- ethyl 1-methylbicyclo[3.1.0]hexane-6-carboxylate

Ethyl diazoacetate (3.42g) was reacted with 1-methylcyclopentene (6.41ml = 5g) using rhodium acetate dimer catalyst (0.1g) as described in Example 12(a). Distillation afforded the title esters (3.50g); $\nu_{max}$ (film) 3050(sh) and 1725$cm^{-1}$; δ ($CDCl_3$, 90MHz) 1.10-1.55, 1.55-2.05 (14H, 2m), and 3.95-4.30 (2H, m); traces of diethyl fumarate and maleate were also observed.

(b) Exo- and endo- 6-(hydroxymethyl)-1-methyl bicyclo[3.1.0]hexane

Exo - and endo - ethyl 1-methylbicyclo[3.1.0]hexane-6-carboxylate (3.50g) was reduced using lithium aluminium hydride (1.16g) as described in Example 9(a). Workup as described therein gave crude product which was purified by silica gel chromatography, eluting with ether-pentane mixtures. Appropriate fractions (t.l.c.) were pooled and evaporated to afford the alcohols (1.79g); $\nu_{max}$ (film) 3600-3100(br) and 3025(sh)$cm^{-1}$; δ($CDCl_3$, 90MHz) 0.80-2.10 (9H, m, 8H on $D_2O$ exch.), 1.23-1.26 (3H, s), and 3.45-3.80 (2H, 2m); exo /endo = ca . 2:3; m/e 126($M^+$) and 108 ($M-H_2O^+$).

(c) Exo- and endo-1-methylbicyclo[3.1.0]hexane-6-carboxaldehyde

Exo - and endo -6-(hydroxymethyl)-1-methylbicyclo[3.1.0]hexane (1.78g) was oxidised using pyridinium chlorochromate (4.56g) as described in Example 1(a). The title aldehyde (1.34g) exhibited $\nu_{max}$ (film) 3.50, 2740, and 1695$cm^{-1}$; δ ($CDCl_3$, 90MHz) 1.10-2.40 (8H, m), 1.30, 1.38 (3H, 2s, ca 2:3), 9.36 and 9.46 (1H, 2d, J s = 6Hz each); m/e 125($MH^+$).

(d) Methyl E,Z-[2-(2-acetamidothiazol-4-yl)-3-(exo-1-methylbicyclo[3.1.0]hex-6-yl)]propenoate

Exo /endo -1-methylbicyclo[3.1.0]hexane-6-carboxaldehyde (1.30g) was condensed with methyl 2-acetamidothiazol-4-acetate (2.25g) as described in Example 1(b). Chromatography (ethyl acetate-hexane mixtures, silica gel) afforded firstly the Z-exo-ester as a solid (0.35g), m.p. 163-165°C (from ethyl acetate-hexane) (Found: C, 60.1; H, 6.3; N, 8.9; S, 9.0; M, 320.1197. $C_{16}H_{20}N_2O_3S$ requires C, 60.0; H, 6.3; N, 8.8; S, 10.0%; M, 320.1195); $\nu_{max}$ (KBr) 1713, 1655, 1608(w), and 1556cm$^{-1}$; $\delta$ ($CDCl_3$, 250MHz) 1.32 (3H, s), 1.20-1.40 (2H, m), 1.55-1.95 (2H, m), 2.19 (1H, dd, J s=11.0Hz and 3.2Hz), 2.23 (3H, s), 3.86 (3H, s), 6.62 (1H, d, J =11.0Hz), 6.96 (1H, s), and 9.77 (1H, brs). The exo -configuration was determined by a nuclear Overhauser study. Further elution of the column afforded the E -ester (0.78g) as a foam, probably the exo -isomer; $\delta$ ($CDCl_3$, 90MHz), inter alia , 6.84 (1H, d, J =10Hz) and 6.96 (1H, s).

(e) Z-[2-(2-Aminothiazol-4-yl)-3-(exo-1-methylbicyclo[3.1.0]hex-6-yl)]propenoic acid

Methyl Z-[2-(2-acetamidothiazol-4-yl)-3-(exo -1-methylbicyclo[3.1.0]hex-6-yl)]propenoate (0.17g) was hydrolysed using 1M NaOH (4.0ml) as described in Example 1(c). The solid obtained on acidification to pH 4.2 was filtered off, washed with a little cold water and dried to afford the title acid (0.10g); $\nu_{max}$ (KBr) 1685-(sh), 1623, 1559, and 1527cm$^{-1}$; $\delta$[$(CD_3)_2SO$, 250MHz) 1.17 (1H, m), 1.25 (3H, s), 1.50-1.85 (6H, m), 1.91 (1H, dd, J s=10.9Hz and 3.1Hz), 6.31 (1H, d, J =10.9Hz), 6.39 (1H, s), and 6.99 (2H, brs, $D_2O$ exch.); m/e 264 ($M^+$) and 246 ($M-H_2O^+$). (Found: M, 264.0932. $C_{13}H_{16}N_2O_2S$ requires M, 264.0932). Further acidification of the aqueous mother liquors to pH2 using 2MHCl followed by extraction with ethyl acetate (3x) afforded additional product (0.04g) which was practically as pure by n.m.r. analysis.

(f) Sodium 6$\beta$-[Z-[2-(2-aminothiazol-4-yl)-3-exo-1-methylbicyclo[3.1.0]hex-6-yl)]propenamido]penicillanate, diastereoisomers

Z-[2-(2-Aminothiazol-4-yl)-3-(exo -1-methylbicyclo[3.1.0]hex-6-yl)]propenoic acid (0.132g) was coupled to 6-aminopenicillanic acid (0.113g) and purified as described in Example 6(d) using NP20SS. The title penicillin (0.061g) showed $\nu_{max}$ (KBr) 1764, 1655(sh), 1609, 1526, and 1457(w)cm$^{-1}$; $\delta$($D_2O$, 250MHz) 1.20-2.00 (8H, m), 1.29, 1.31 (3H, 2s), 1.53, 1.64 (6H, 2s), 4.25 (1H, s), 5.50-5.70 (2H, m), 6.16 (1H, d, J =10.8Hz), and 6.42 (1H, s); m/e 507 ($MNa^+$), 485 ($MH^+$), and 463 ($MH^+$, free acid).

Example 16

Also prepared by methods similar to those described above was sodium 6$\beta$-[Z-[2-(2-aminothiazol-4-yl)-3-(spiro[2,4]hept-4-yl)]propenamido]penicillanate.

## Claims

1. A compound of formula (I) or a pharmaceutically acceptable salt or in -vivo hydrolysable ester thereof:

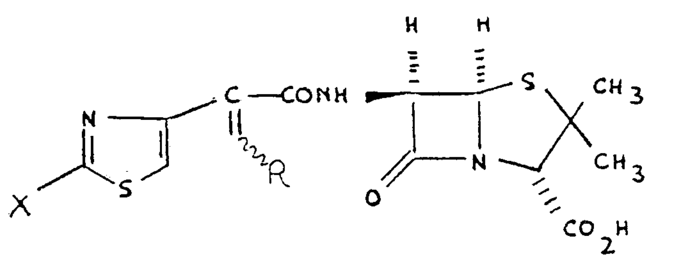

(I)

wherein X is hydrogen or a group $NHR^1$, wherein $R^1$ is hydrogen or an amino protecting group, and R is an optionally substituted spiro, fused or bridged bicyclic group optionally containing one or more heteroatoms selected from oxygen, nitrogen and sulphur.

2. A compound according to claim 1, wherein R is selected from the group consisting of: bicyclo[2.2.1]hept-2-yl,3-methyl bicyclo[2.2.1]hept-2-yl, bicyclo[2.2.1]hept-5-en-2-yl, bicyclo[4.4.0]dec-2-yl, bicyclo[4.3.0]non-7-yl, endo-7-oxabicyclo[2.2.1]hept-2-yl, exo-bicyclo[4.1.0]hept-7-yl, exo-bicyclo[3.1.0]hex-6-yl, endo-1-methoxybicyclo[2.2.2]oct-5-ene-2-yl, exo-1-methoxybicyclo[2.2.2]oct-5-ene-2-yl, cis -bicyclo[3.3.0]oct-2-yl, exo-bicyclo[2.2.2]oct-5-ene-2-yl, exo-3-oxabicyclo[3.1.0]hex-6-yl, endo-3-oxabicyclo[3.1.0]hex-6-yl, exo-bicyclo[4.1.0]hept-3-ene-7-yl, endo-bicyclo[4.1.0]hept-3-ene-7-yl, exo-bicyclo[5.1.0]oct-8-yl, exo-1-methylbicyclo[3.1.0]hex-6-yl, and spiro[2,4]hept-4-yl.

3. A compound according to claim 1 or 2, which is in the form of the syn isomer.

4. A compound selected from the group consisting of 6β-[Z-[2-(2-aminothiazol-4-yl)-3-(bicyclo[2.2.1]hept-2-yl)]-propenamido]penicillanic acid,
6β-[Z-[2-(2-aminothiazol-4-yl)-3-(3-methylbicyclo[2.2.1]hept-2-yl)]propenamido]penicillanic acid,
6β-[Z-[2-(2-aminothiazol-4- yl)-3-(bicyclo[2.2.1]hept-5-en-2-yl)]propenamido]penicillanic acid,
6β-[Z-[2-(2-aminothiazol-4-yl)-3-(bicyclo[4.4.0]dec-2-yl)]propenamido]penicillanic acid,
6β-[Z-[2-(2-aminothiazol-4-yl)-3-(bicyclo[4.3.0]non-7-yl)]propenamido]penicillanic acid,
6β-[Z-[2-(2-aminothiazol-4-yl)-3-(endo-7-oxabicyclo[2.2.1]hept-2-yl)]propenamido]penicillanic acid,
6β-[Z-[2-(2-aminothiazol-4-yl)-3-(exo-bicyclo[4.1.0]hept-7-yl)]propenamido]penicillanic acid,
6β-[Z-[2-(2-aminothiazol-4-yl)-3-(exo-bicyclo[3.1.0]hex-6-yl)]propenamido]penicillanic acid,
6β-[Z-[2-(2-aminothiazol-4-yl)-3-(endo-1-methoxybicyclo[2.2.2]oct-5-ene-2- yl)]propenamido]penicillanic acid,
6β-[Z-[2-(2-aminothiazol-4-yl)-3-(exo-1-methoxybicyclo[2.2.2]oct-5-ene-2-yl)]propenamido]penicillanic acid,
6β-[Z-[2-(2-aminothiazol-4-yl)-3-(cis-bicyclo[3.3.0]oct-2-yl)]propenamido]penicillanic acid,
6β-[Z-[2-(2-aminothiazol-4-yl)-3-(exo-bicyclo[2.2.2]oct-5-ene-2-yl)]propenamido]penicillanic acid,
6β-[Z-[2-(2-aminothiazol-4-yl)-3-(exo-3-oxabicyclo[3.1.0]hex-6-yl)]propenamido]penicillanic acid,
6β-[Z-[2-(2-aminothiazol-4-yl)-3-(endo-3-oxabicyclo[3.1.0]hex-6-yl)]propenamido]penicillanic acid,
6β-[2-(2-aminothiazol-4-yl)-3-(exo-bicyclo[4.1.0]hept-3-ene-7-yl)]propenamido]penicillanic acid,
6β-[Z-[2-(2-aminothiazol-4-yl)-3-(endo-bicyclo[4.1.0]hept-3-ene-7-yl)]propenamido]penicillanic acid,
6β-[Z-[2-(2-aminothiazol-4-yl)-3-(exo-bicyclo[5.1.0]oct-8-yl)]propenamido]penicillanic acid,
6β-[Z-[2-(2-aminothiazol-4-yl)-3-(exo-1-methylbicyclo[3.1.0]hex-6-yl)]propenamido]pencillanic acid, and
6β-[Z-[2-(2-aminothiazol-4-yl)-3-(spiro[2,4]hept-4-yl)]propenamido]pencillanic acid, and pharmaceutically acceptable salts and in -vivo hydrolysable esters thereof.

5. A process for the preparation of a compound according to any preceding claim, which process comprises treating a compound of formula (II) or salt thereof:

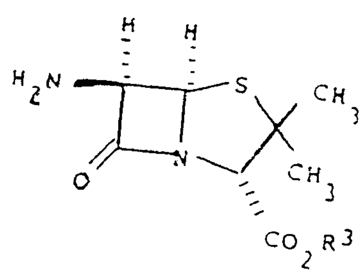

(II)

wherein the amino group is optionally substituted with a group which permits acylation to take place, and $R^3$ is hydrogen or a readily removable carboxyl blocking group; with an acylating agent derived from the acid of formula (III):

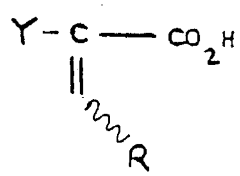

(III)

wherein Y is a group of formula:

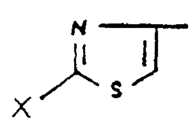

or a group which is convertable thereto, and R and X are as defined with respect to formula (I).
6. A pharmaceutical composition which comprises a compound according to any one of claims 1 to 4 together with a pharmaceutically acceptable carrier.
7. A compound according to any one of claims 1 to 4, for use in therapy.
8. Use of a compound according to any one of claims 1 to 4 in the manufacture of a medicament for use in the treatment of bacterial infections in humans or animals.

Claims for the following Contracting State: ES.

1. A process for the preparation of a compound of formula (I) or a pharmaceutically acceptable salt or in-vivo hydrolysable ester thereof:

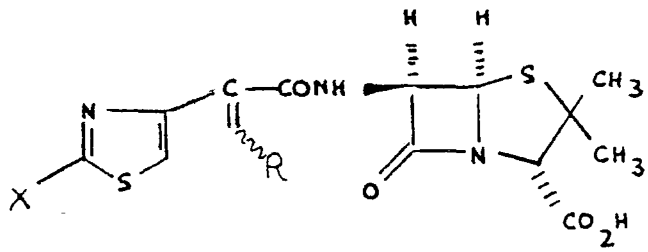

(I)

wherein X is hydrogen or a group $NHR^1$, wherein $R^1$ is hydrogen or an amino protecting group, and R is an optionally substituted spiro, fused or bridged bicyclic group optionally containing one or more heteroatoms selected from oxygen, nitrogen and sulphur, which process comprises treating a compound of formula (II) or salt thereof:

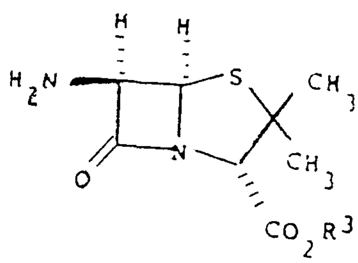

(II)

wherein the amino group is optionally substituted with a group which permits acylation to take place, and $R^3$ is hydrogen or a readily removable carboxyl blocking group; with an acylating agent derived from the acid of formula (III):

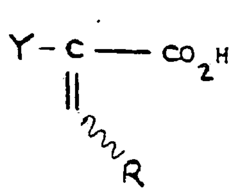

(III)

wherein Y is a group of formula:

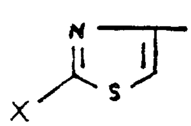

or a group which is convertable thereto, and R and X are as defined with respect to formula (I).

2. A process according to claim 1, wherein R is selected from the group consisting of: bicyclo[2.2.1]hept-2-yl,3-methyl bicyclo[2.2.1]hept-2-yl, bicyclo[2.2.1]hept-5-en-2-yl, bicyclo[4.4.0]dec-2-yl, bicyclo[4.3.0]non-7-yl, endo-7-oxabicyclo[2.2.1]hept-2-yl, exo-bicyclo[4.1.0]hept-7-yl, exo-bicyclo[3.1.0]hex-6-yl, endo-1-methoxybicyclo[2.2.2]oct-5-ene-2-yl, exo-1-methoxybicyclo[2.2.2]oct-5-ene-2-yl, cis -bicyclo[3.3.0]oct-2-yl, exo-bicyclo[2.2.2]oct-5-ene-2-yl, exo-3-oxa-bicyclo[3.1.0]hex-6-yl, endo-3-oxabicyclo[3.1.0]hex-6-yl, exo-bicyclo[4.1.0]hept-3-ene-7-yl, endo-bicyclo[4.1.0]hept-3-ene-7-yl, exo-bicyclo[5.1.0]oct-8-yl, exo-1-methylbicyclo[3.1.0]hex-6-yl, and spiro[2,4]hept-4-yl.

3. A process according to claim 1 or 2, for the preparation of a syn isomer.

4. A process according to any preceding claim, for the preparation of

6β-[Z-[2-(2-aminothiazol-4-yl)-3-(bicyclo[2.2.1]hept-2-yl)]-propenamido]penicillanic acid, or a pharmaceutically acceptable salt or in -vivo hydrolysable ester thereof,

6β-[Z-[2-(2-aminothiazol-4-yl)-3-(3-methylbicyclo[2.2.1]hept-2-yl)]propenamido]penicillanic acid, or a pharmaceutically acceptable salt or in -vivo hydrolysable ester thereof,

6β-[Z-[2-(2-aminothiazol-4- yl)-3-(bicyclo[2.2.1]hept-5-en-2-yl)]propenamido]penicillanic acid, or a pharmaceutically acceptable salt or in -vivo hydrolysable ester thereof,

6β-[Z-[2-(2-aminothiazol-4-yl)-3-(bicyclo[4.4.0]dec-2-yl)]propenamido]penicillanic acid, or a pharmaceutically acceptable salt or in -vivo hydrolysable ester thereof,

6β-[Z-[2-(2-aminothiazol-4-yl)-3-(bicyclo[4.3.0]non-7-yl)]propenamido]penicillanic acid, or a pharmaceutically acceptable salt or in -vivo hydrolysable ester thereof,

6β-[Z-[2-(2-aminothiazol-4-yl)-3-(endo-7-oxabicyclo[2.2.1]hept-2-yl)]propenamido]penicillanic acid, or a pharmaceutically acceptable salt or in -vivo hydrolysable ester thereof,

6β-[Z-[2-(2-aminothiazol-4-yl)-3-(exo-bicyclo[4.1.0] hept-7-yl)]propenamido]penicillanic acid, or a pharmaceutically acceptable salt or in -vivo hydrolysable ester thereof,

6β-[Z-[2-(2-aminothiazol-4-yl)-3-(exo-bicyclo[3.1.0]hex-6-yl)]propenamido]penicillanic acid. or a pharmaceutically acceptable salt or in -vivo hydrolysable ester thereof,

6β-[Z-[2-(2-aminothiazol-4-yl)-3-(endo-1-methoxybicyclo[2.2.2]oct-5-ene-2-yl)]propenamido] penicillanic acid, or a pharmaceutically acceptable salt or in -vivo hydrolysable ester thereof,

6β-[Z-[2-(2-aminothiazol-4-yl)-3-(exo-1-methoxybicyclo[2.2.2]oct-5-ene-2-yl)]propenamido] penicillanic acid, or a pharmaceutically acceptable salt or in -vivo hydrolysable ester thereof,

6β-[Z-[2-(2-aminothiazol-4-yl)-3-(cis-bicyclo[3.3.0]oct-2-yl)]propenamido]penicillanic acid, or a pharmaceutically acceptable salt or in -vivo hydrolysable ester thereof,

6β-[Z-[2-(2-aminothiazol-4-yl)-3-(exo-bicyclo[2.2.2]oct-5-ene-2-yl)]propenamido]penicillanic acid, or a pharmaceutically acceptable salt or in -vivo hydrolysable ester thereof,

6β-[Z-[2-(2-aminothiazol-4-yl)-3-(exo-3-oxabicyclo[3.1.0]hex-6-yl)]propenamido]penicillanic acid, or a pharmaceutically acceptable salt or in -vivo hydrolysable ester thereof,

6β-[Z-[2-(2-aminothiazol-4-yl)-3-(endo-3-oxabicyclo[3.1.0]hex-6-yl)]propenamido]penicillanic acid, or a pharmaceutically acceptable salt or in -vivo hydrolysable ester thereof,

6β-[2-(2-aminothiazol-4-yl)-3-(exo-bicyclo[4.1.0]hept-3-ene-7yl)]propenamido]penicillanic acid, or a pharmaceutically acceptable salt or in -vivo hydrolysable ester thereof,

6β-[Z-[2-(2-aminothiazol-4-yl)-3-(endo-bicyclo[4.1.0]hept-3-ene-7-yl)]propenamido]penicillanic acid, or a pharmaceutically acceptable salt or in -vivo hydrolysable ester thereof,

6β-[Z-[2-(2-aminothiazol-4-yl)-3-(exo-bicyclo[5.1.0]oct-8-yl)]propenamido]penicillanic acid, or a pharmaceutically acceptable salt or in -vivo hydrolysable ester thereof,

6β-[Z-[2-(2-aminothiazol-4-yl)-3-(exo-1-methylbicyclo[3.1.0]hex-6-yl)]propenamido]pencillanic acid, or a pharmaceutically acceptable salt or in -vivo hydrolysable ester thereof, or

6β-[Z-[2-(2-aminothiazol-4-yl)-3-(spiro[2,4]hept-4-yl)]propenamido]pencillanic acid, or a pharmaceutically acceptable salt or in -vivo hydrolysable ester thereof.

5. A pharmaceutical composition which comprises a compound of formula (I) or a pharmaceutically acceptable salt or in -vivo hydrolysable ester thereof together with a pharmaceutically acceptable carrier.

6. A compound of formula (I) or a pharmaceutically acceptable salt or in -vivo hydrolysable ester thereof for use in therapy.

7. Use of a compound of formula (I) or a pharmaceutically acceptable salt or in -vivo hydrolysable ester thereof in the manufacture of a medicament for use in the treatment of bacterial infections in humans or animals.